# EUROPÄISCHE PATENTSCHRIFT

(11) **EP 3 651 609 B1**
(45) Veröffentlichungstag und Bekanntmachung des Hinweises auf die Patenterteilung: **05.01.2022**
(21) Anmeldenummer: 18749303.6
(22) Anmeldetag: 02.07.2018
(51) Int. Cl.: A45D 1/00, A45D 6/20, A61B 5/00, A45D 44/00, A45D 6/00

(54) **HAARBEHANDLUNGSSYSTEM UND VERFAHREN ZUM KOSMETISCHEN BEHANDELN VON HAAREN**
HAIR TREATMENT SYSTEM AND METHOD FOR COSMETICALLY TREATING HAIR
SYSTÈME DE TRAITEMENT CAPILLAIRE ET PROCÉDÉ DE TRAITEMENT COSMÉTIQUE DES CHEVEUX

(30) Priorität: 10.07.2017 DE 102017211781
(43) Veröffentlichungstag der Anmeldung: 20.05.2020
(73) Patentinhaber: Henkel AG & Co. KGaA, 40589 Düsseldorf (DE)
(72) Erfinder: KUMPAN-BAHRAMI, Esther, 40547 Düsseldorf (DE); KNUEBEL, Hans Georg, 40219 Düsseldorf (DE)
(86) Internationale Anmeldenummer: PCT/EP2018/067829
(87) Internationale Veröffentlichungsnummer: WO 2019/011700

(56) Entgegenhaltungen:
- WO-A1-2018/219899
- JP-A- 2014 200 423
- US-A1- 2012 272 993
- US-A1- 2012 312 320

## Beschreibung

Die Erfindung betrifft Haarkosmetik, insbesondere ein Haarbehandlungssystem und ein Verfahren zum kosmetischen Behandeln von Haaren.

Nutzer (auch als Verbraucher bezeichnet) wissen oft nicht, in welchem Maße ihre Haare geschädigt sind, und wissen auch sonst oft wenig über den Zustand ihrer Haare. Der Haarzustand, und insbesondere der Haarschädigungsgrad, kann je nach Behandlungshistorie und Veranlagung stark variieren. Diese Information kann wichtig sein, damit ein/e richtige/s, d.h. zum Haarschädigungsgrad passende/s kosmetische Haarbehandlung und/oder ein kosmetisches Haarbehandlungsmittel ausgewählt werden kann/können.

Bei einer semipermanenten Haarverformung, welche beispielsweise mittels eines Glätteisens und/oder eines Lockenstabs oder einer ähnlichen Vorrichtung herbeiführbar ist, können (z.B. chemische) Haarbehandlungsmittel verwendet werden, welche eine Wirkung der mechanischen Vorrichtungen, welche für die Haarverformung genutzt werden, verstärken können. Glätteisen, Lockenstab und ähnliche Vorrichtungen werden hierin auch verallgemeinernd als Kleinelektrogeräte bezeichnet.

Bei der semipermanenten Haarverformung wird zur Erzielung des kosmetischen Effekts in die Haarstruktur eingegriffen. Dies geschieht beispielsweise durch ein Erhitzen des Haares beim Verwenden des Glätteisens oder des Lockenstabs. Dieser Vorgang kann aufgrund der einwirkenden hohen Temperaturen (120°C bis 240°C) für das Haar möglicherweise nicht unschädlich sein. Insbesondere vorgeschädigtes Haar kann bei einer falsch angewendeten semipermanenten Haarverformung, beispielsweise bei zu starkem Hitzeeintrag durch ein Glätteisen stark (weiter) geschädigt werden.

Eine genaue Abstimmung der bei der Glättung eingesetzten Temperaturen und/oder kosmetischen Pflegemittel kann daher bei der semipermanenten Haarverformung eine große Bedeutung haben.

Insbesondere Laien kann es an Erfahrung fehlen, wie eine semipermanente Haarumformung mittels Kleinelektrogeräten (zum Beispiel ein Glätten mittels eines Glätteisens oder ein Locken des Haares mittels eines Lockenstabs) zu erreichen ist, ohne das Haar zu schädigen. Außerdem kann es ihnen an Erfahrung fehlen, wie unter gegebenen klimatischen Bedingungen ein optimales Ergebnis zu erzielen ist.

Eine effektive Haarumformung kann meist erst bei Temperaturen von ca. 190°C oder mehr erzielt werden. Ab dieser Temperatur können Haarschäden beobachtet werden.

Es besteht somit ein Bedarf an einem Haarbehandlungsverfahren oder einem Haarbehandlungssystem, welches eine schonende Behandlung des Haars ermöglicht. Außerdem wäre es wünschenswert, dem Nutzer während einer kosmetischen Haarbehandlung eine objektive Beurteilung eines Behandlungserfolgs und/oder eines Behandlungsverlaufs zu ermöglichen.

Dokument JP2014200423 A beschreibt ein Verfahren und ein System zur Behandlung von Haaren, bei dem der pH-Wert des Haares gemessen wird und abhängig davon eine bestimmte Menge eines sauren Additivs zugegeben wird, um das Haar geschmeidig zu halten.

In verschiedenen Ausführungsbeispielen kann das Haarbehandlungssystem über einen Sensor verfügen, dessen Messergebnis berücksichtigt bei einem Ausführen der Haarbehandlung wird.

In verschiedenen Ausführungsbeispielen wird ein ganzheitliches Ökosystem aus intelligenten/smarten Geräten bereitgestellt, welche von einer Problemidentifikation (Analyse) über eine Beratung (Auswertung) bis zu einer endgültigen Bereitstellung eines gewünschten Nutzens (smartes Kleinelektrogerät, gegebenenfalls in Kombination mit geeignetem gegebenenfalls individualisiertem Produkt) beispielsweise eine verbesserte Haarqualität und/oder ein verbessertes Styling bereitstellen.

In verschiedenen Ausführungsbeispielen kann ein Haarbehandlungssystem bereitgestellt werden, beispielsweise ein Haarumformungssystem. Das Haarbehandlungssystem kann in verschiedenen Ausführungsbeispielen eine Sensorvorrichtung (auch als Sensoreinheit, Analysevorrichtung oder Analyseeinheit bezeichnet) und eine Haarbehandlungsvorrichtung aufweisen.

Die Sensorvorrichtung kann in verschiedenen Ausführungsbeispielen einen Haarstatus, auch als Haarzustand bezeichnet, ermitteln. Der Haarzustand kann einen oder mehrere Haarzustandsparameter aufweisen.

Die Sensorvorrichtung kann in verschiedenen Ausführungsbeispielen ein Smartphone, ein Haarband, einen Kamm oder Ähnliches aufweisen. Die Sensorvorrichtung kann in verschiedenen Ausführungsbeispielen mit mindestens einem Sensor ausgestattet sein. Der Sensor kann in verschiedenen Ausführungsbeispielen eine Kamera, beispielsweise in Verbindung mit einem Mikroskop, einen Nahinfrarotsensor, einen VIS-/NIR-Sensor (d.h. einen Sensor, der in einem Wellenlängenbereich empfindlich ist, der vom Bereich sichtbaren Lichts (VIS) bis zum Nahinfrarotbereich (NIR) reicht), einen Sensor zum Ermitteln von Umweltbedingungen, wie beispielsweise einen Luftfeuchtesensor, einen Temperatursensor, ein Windsensor, usw. umfassen.Die Sensoren, welche in verschiedenen Ausführungsbeispielen zum Ermitteln eines Zustands vor, während und/oder nach der Haarbehandlung genutzt werden, und deren Messergebnisse eine Grundlage dafür bilden, wie die Haarbehandlung ausgeführt wird, können hierin auch als Zustandssensoren bezeichnet werden.

In verschiedenen Ausführungsbeispielen kann die Haarbehandlungsvorrichtung eingerichtet sein, ein Haarbehandlungsmittel auf das Haar aufzubringen. Dafür kann die Haarbehandlungsvorrichtung beispielsweise mit einer integrierten Kartusche ausgerüstet sein. In der Kartusche kann das Haarbehandlungsmittel aufgenommen sein und mittels einer Dosiervorrichtung dosiert auf das Haar aufbringbar sein, beispielsweise mittels einer Sprühvorrichtung und/oder einer Tropfvorrichtung.

Die Haarbehandlungsvorrichtung kann in verschiedenen Ausführungsbeispielen zum Applizieren eines kosmetischen Mittels und zum Anwenden mechanischer Arbeit, zum Beispiel zum Drehen oder Dehnen einer Haarsträhne, und/oder zu einem Anwenden von Temperatur, zum Beispiel als ein Eintrag von Wärme und/oder Kälte ins Haar, eingerichtet sein.

In verschiedenen Ausführungsbeispielen können die Analyseeinheit und das Kleinelektrogerät separat ausgeführt sein.

In verschiedenen Ausführungsbeispielen können die Analyseeinheit und das Kleinelektrogerät integriert ausgeführt sein.

In verschiedenen Ausführungsbeispielen können eine Intensität der mechanischen Arbeit und/oder der Temperatur in Abhängigkeit von mittels der Analysevorrichtung übermittelten/ermittelten Daten geregelt werden.

In verschiedenen Ausführungsbeispielen kann das Haarbehandlungssystem ferner, beispielsweise als ein zusätzliches Element, eine Eingabevorrichtung und/oder eine Ausgabevorrichtung aufweisen.

Die Eingabevorrichtung kann in verschiedenen Ausführungsbeispielen eine haptische und/oder eine akustische Eingabevorrichtung sein.

Die Ausgabevorrichtung kann in verschiedenen Ausführungsbeispielen eine optische und/oder eine akustische Ausgabevorrichtung sein.

Die Eingabevorrichtung kann in verschiedenen Ausführungsbeispielen genutzt werden zum Eingeben eines Steuerbefehls (z.B. Starten, Ändern, Stoppen des Haarbehandlungssystems und/oder von Teilen des Haarbehandlungssystems, z.B. nur von der Haarbehandlungsvorrichtung oder nur von der Sensorvorrichtung).

Die Ausgabevorrichtung kann in verschiedenen Ausführungsbeispielen für ein Ausgeben eines Analyseergebnisses und/oder ein Ausgeben einer Verfahrens-und/oder Produktempfehlung und/oder für ein Ausgeben eines Behandlungsfortschritts genutzt werden.

In verschiedenen Ausführungsbeispielen kann die Eingabe-und/oder Ausgabevorrichtung separat ausgeführt sein, d.h. separat von der Haarbehandlungsvorrichtung und von der Analysevorrichtung.

In verschiedenen Ausführungsbeispielen kann die Eingabevorrichtung einen berührungsempfindlichen Bildschirm, eine Tastatur, ein Mikrofon, eine Maus oder Ähnliches aufweisen, beispielsweise als Teil eines Smartphones, Tablets, Laptops, Smart Mirrors oder Ähnliches.

In verschiedenen Ausführungsbeispielen kann die Ausgabevorrichtung einen (z.B. berührungsempfindlichen) Bildschirm, einen Lautsprecher oder Ähnliches aufweisen, beispielsweise als Teil eines Smartphones, Tablets, Laptops, Smart Mirrors oder Ähnliches.

In verschiedenen Ausführungsbeispielen kann die Eingabe- und/oder Ausgabevorrichtung einen berührungsempfindlichen Bildschirm, eine Kombination aus Bildschirm und Tastatur oder eine Kombination aus Mikrofon und/oder Lautsprecher aufweisen.

In verschiedenen Ausführungsbeispielen kann die Eingabe-und/oder Ausgabevorrichtung in die Sensorvorrichtung integriert ausgeführt sein, beispielsweise als berührungsempfindlicher Bildschirm, als eine Kombination aus Bildschirm und Tastatur, als ein Mikrofon und/oder ein Lautsprecher.

In verschiedenen Ausführungsbeispielen kann die Eingabe-und/oder Ausgabevorrichtung in die Haarbehandlungsvorrichtung, z.B. das Kleinelektrogerät, integriert ausgeführt sein, beispielsweise als berührungsempfindlicher Bildschirm oder als eine Kombination aus Bildschirm und Tastatur, als ein Mikrofon und/oder als ein Lautsprecher.

Das Haarbehandlungsmittel, welches auf das Haar aufgebracht werden kann, kann in verschiedenen Ausführungsbeispielen ein handelsübliches Produkt sein.

In verschiedenen Ausführungsbeispielen kann das Haarbehandlungsmittel ein individualisiertes Produkt sein, beispielsweise ein individualisiertes Produkt, welches nach einer (z.B. erstmaligen) Ermittlung des mindestens einen Haarzustandsparameters (d.h. nach einer Analyse des Haars, z.B. mittels der Sensorvorrichtung) hergestellt und mitgegeben oder versendet werden kann.

In verschiedenen Ausführungsbeispielen kann das individualisierte Produkt nach der Ermittlung des Haarzustandsparameters zu Hause hergestellt werden, beispielsweise mittels einer in das Haarbehandlungssystem integrierten Mischvorrichtung für Pflege- und/oder Stylingmittel.

In verschiedenen Ausführungsbeispielen kann die Mischvorrichtung separat bereitgestellt sein oder werden, d.h. physisch getrennt von der Haarbehandlungsvorrichtung, von der Sensorvorrichtung und von der Eingabe- und/oder Ausgabevorrichtung ausgeführt sein.

In verschiedenen Ausführungsbeispielen kann die Mischvorrichtung in das Kleinelektrogerät integriert sein.

In verschiedenen Ausführungsbeispielen kann die Mischvorrichtung in die separate Eingabe-und/oder Ausgabevorrichtung integriert sein.

In verschiedenen Ausführungsbeispielen kann eine Umformungswirkung durch eine additive Wirkung von Pflege- und/oder Stylingmittel und Kleinelektrogerät (d.h. die mechanische Einwirkung und/oder Wärmeeinwirkung durch die Haarbehandlungsvorrichtung) erzielt oder gefördert werden. Anders ausgedrückt kann die Umformungswirkung durch die synergetische Wirkung von Pflege-und/oder Stylingmittel und Kleinelektrogerät (d.h. die mechanische Einwirkung und/oder Wärmeeinwirkung durch die Haarbehandlungsvorrichtung) erzielt oder gefördert werden.

Bei einer separaten Ausführung von Sensorvorrichtung, Haarbehandlungsvorrichtung, Ein-/Ausgabevorrichtung und/oder Mischvorrichtung kann in verschiedenen Ausführungsbeispielen eine Kommunikation innerhalb des mehrteiligen Systems kabellos erfolgen.

Die kabellose Kommunikation kann in verschiedenen Ausführungsbeispielen mittels eines zentralen Kommunikationsknotenpunkts bereitgestellt werden.

In verschiedenen Ausführungsbeispielen kann die Kommunikation innerhalb einer aus den einzelnen Komponenten des Haarbehandlungssystems entsprechend einer Verfahrensabfolge gebildeten Kommunikationsreihe erfolgen.

In verschiedenen Ausführungsbeispielen kann die Kommunikation innerhalb eines aus den Komponenten des Haarbehandlungssystems gebildeten Netzwerks erfolgen.

In verschiedenen Ausführungsbeispielen wird ein ganzheitliches System bereitgestellt, welches eine standardisierte und objektive Beurteilung eines Behandlungsergebnisses ermöglicht. In verschiedenen Ausführungsbeispielen kann unter Zuhilfenahme eines breiten Daten- und Erfahrungssatzes (welcher beispielsweise mittels Zugreifens des Haarbehandlungssystems auf eine Prozessor-Cloud-Architektur (kurz: Cloud) dem Haarbehandlungssystem zugänglich gemacht sein oder werden kann) eine Optimierung eines Behandlungsergebnisses ermöglicht werden.

In verschiedenen Ausführungsbeispielen kann das Haarbehandlungssystem als ein lernendes System ausgebildet sein. Dafür können beispielsweise Erfahrungen anderer Nutzer fortlaufend dem Haarbehandlungssystem bereitgestellt werden, beispielsweise mittels Bereitstellens der Erfahrungen durch die anderen Nutzer an die Cloud und ggf. Weiterverarbeitens der Erfahrungen in der Cloud.

In verschiedenen Ausführungsbeispielen können ein Verfahren und eine Vorrichtung bereitgestellt werden, welche eine effektive Haarumformung bei Temperaturen unter 190°C ermöglichen.

In verschiedenen Ausführungsbeispielen kann bei einer semipermanenten Umformung der Haare eine Abgabe von Pflege- und/oder Stylingmitteln und eine Erhitzung der Haare in einem Verfahrensschritt vereint werden.

Im Bereich der semipermanenten Haarverformung werden in verschiedenen Ausführungsbeispielen Verfahren unter Verwendung stromführender Kleingeräte wie Glätteisen oder Lockenstäbe bereitgestellt, sowie die dafür geeigneten Haarbehandlungsvorrichtungen und Haarbehandlungssysteme.

In verschiedenen Ausführungsbeispielen wird eine Vorrichtung bereitgestellt, welche eine Kombination aus einem Stylinggerät (z.B. Lockenstab, Glätteisen oder Ähnliches), einer Sensorik, d.h. mindestens einen Sensor (wobei der mindestens eine Sensor in das Stylinggerät integriert oder physisch getrennt vom Stylinggerät, z.B. Teil einer separaten Sensorvorrichtung, sein kann), einer elektronischen Schaltkreisvorrichtung (z.B. einem Prozessor, z.B. einem Mikroprozessor) und einem Aktuator (zum Steuern oder Regeln einer Haarbehandlungsfunktionalität des Stylinggeräts, z.B. eine Temperatursteuerung, ein Steuern eines Anpressdrucks und/oder ein Dosieren eines Haarbehandlungsmittels) aufweist. In verschiedenen Ausführungsbeispielen kann die Kombination ferner eine Ein- und/oder Ausgabevorrichtung und/oder eine Mischvorrichtung aufweisen.

Ferner wird in verschiedenen Ausführungsbeispielen ein Verfahren bereitgestellt, welches diese Vorrichtung nutzt.

In verschiedenen Ausführungsbeispielen kann die elektronische Schaltkreisvorrichtung eingerichtet sein, Sensordaten vom Sensor zu empfangen, die Sensordaten auszuwerten und gegebenenfalls die ausgewerteten Sensordaten mit mindestens einer externen Datenbank (z.B. mittels einer Cloud) abzugleichen.

In verschiedenen Ausführungsbeispielen kann der Prozessor ferner eingerichtet sein, aus den ausgewerteten Sensordaten Handlungsanweisungen abzuleiten (z.B. ein Verfahrensprofil einschließlich Temperatur und/oder eingesetzten Pflege- und/oder Stylingmitteln).

In verschiedenen Ausführungsbeispielen kann die elektronische Schaltkreisvorrichtung ferner eingerichtet sein, gegebenenfalls eine Handlungsanweisung an den Aktuator zu übermitteln, wobei der Aktuator Teil der Haarbehandlungsvorrichtung oder des Haarbehandlungssystems sein kann.

In verschiedenen Ausführungsbeispielen kann der Aktuator die von der elektronischen Schaltkreisvorrichtung bereitgestellte Handlungsanweisung umsetzen. Eine vom Aktuator ausgeführte Handlung kann in verschiedenen Ausführungsbeispielen eine mechanische Wirkung haben (z.B. als Steuern oder Regeln eines Anpressdrucks eines Glätteisens oder eines Lockenstabs und/oder eine automatische Einstellung einer Dosiervorrichtung für ein Haarbehandlungsmittel am Stylinggerät), und/oder der Aktuator kann eingerichtet sein, eine automatische Temperatureinstellung zu bewirken.

In verschiedenen Ausführungsbeispielen kann der Sensor einen Haarschädigungssensor aufweisen. Der Haarschädigungssensor kann in verschiedenen Ausführungsbeispielen eingerichtet sein, mittels einer Nahinfrarot-Spektroskopie und/oder einer Fluoreszenzspektroskopie einen Aminosäureoxidationsproduktgehalt, insbesondere einen Cysteinsäuregehalt, des Haars zu ermitteln und daraus einen Haarschädigungsgrad des Haars zu ermitteln. Der Haarschädigungssensor kann in verschiedenen Ausführungsbeispielen eingerichtet sein, mittels einer Nahinfrarot-Spektroskopie einen Gehalt an einer oder mehreren Aminosäure(n) im Haar zu ermitteln und daraus einen Haarschädigungsgrad des Haars zu ermitteln. Der Haarschädigungssensor kann in verschiedenen Ausführungsbeispielen eingerichtet sein, während eines Kämmens des Haars erfasste akustische Emissionen aufzunehmen und anhand dessen den Haarschädigungsgrad des Haars zu ermitteln, ggf. unter Zuhilfenahme des Prozessors.

In verschiedenen Ausführungsbeispielen kann der Haarschädigungssensor einen mikroskopischen Fotosensor aufweisen. Der mikroskopische Fotosensor kann eingerichtet sein, eine Haaroberflächenrauigkeit zu ermitteln oder das Ermitteln der Haaroberflächenrauigkeit zu ermöglichen.

In verschiedenen Ausführungsbeispielen kann der Haarschädigungssensor eine Kamera aufweisen, die an ein Interferenzreflexionsmikroskop der Sensorvorrichtung gekoppelt sein kann.

In verschiedenen Ausführungsbeispielen kann der Sensor einen Haardickesensor aufweisen. Der Haardickesensor kann in verschiedenen Ausführungsbeispielen eingerichtet sein, mittels eines Lichtsensors eine Haardicke zu ermitteln. Beispielsweise kann bei dem Ermitteln der Haardicke berücksichtigt werden, dass dickeres Haar mehr Licht absorbiert. Der Haardickesensor kann dafür in verschiedenen Ausführungsbeispielen derart eingerichtet sein, dass in ein vorgegebenes Volumen eine vorbestimmte Menge an Haar, z.B. einlagig, einbringbar ist, und das Volumen von Licht mit einer vorbestimmten Intensität bestrahlt wird, wobei die Lichtmenge, die nach dem Durchstrahlen des Haars den Sensor erreicht, mittels des Sensors gemessen werden kann. Mittels des Haardickesensors kann, ggf. in Verbindung mit der elektronischen Schaltkreisvorrichtung, z.B. dem Prozessor, anhand des erfassten Lichts die Haardicke ermittelt werden.

In verschiedenen Ausführungsbeispielen kann der Haardickesensor, z.B. in einem Fall, dass der Haardickesensor eine Farbkamera aufweist, außerdem für ein Ermitteln einer Haarfarbe genutzt werden, ggf. unter Zuhilfenahme der elektronischen Schaltkreisvorrichtung.

In verschiedenen Ausführungsbeispielen kann der Haardickesensor einen Ultraschallsensor aufweisen. Der Ultraschallsensor kann eingerichtet sein, Ultraschallwellen in Richtung zu den Haaren abzustrahlen, von den Haaren reflektierte Ultraschallwellen zu erfassen und daraus, ggf. in Verbindung mit dem Prozessor, die Haardicke zu ermitteln.

In verschiedenen Ausführungsbeispielen kann der mindestens eine Sensor einen Haarlängesensor aufweisen. Der Haarlängesensor kann beispielsweise mindestens einen Lagesensor aufweisen, der eine Bestimmung einer Wegstrecke ermöglicht, welche im Haar zurückgelegt wurde. In verschiedenen Ausführungsbeispielen kann der Haarlängesensor kombiniert sein mit einem Sensor zum Berechnen einer Kämmbarkeit des Haars (siehe unten).

In verschiedenen Ausführungsbeispielen kann anstelle eines Ermittelns der Haarlänge mittels des Haarlängesensors die Haarlänge vom Nutzer bereitgestellt werden oder sein. Beispielsweise kann der Nutzer die Haarlänge selbst ausmessen und den gemessenen Haarlängewert der Haarbehandlungsvorrichtung oder dem Haarbehandlungssystem bereitstellen.

In verschiedenen Ausführungsbeispielen kann der Haardickesensor einen photooptischen Sensor aufweisen, bei welchem ein Bild von mindestens einem Haar aufgenommen wird.

In verschiedenen Ausführungsbeispielen kann der Haardickesensor einen thermischen Sensor aufweisen.

In verschiedenen Ausführungsbeispielen kann der Sensor einen Glätte-/Lockigkeitssensor aufweisen, der eingerichtet sein kann, eine Haarstruktur im Sinne glatten Haars bis hin zu lockigem oder krausem Haar zu ermitteln. Der Glätte-/Lockigkeitssensor kann in verschiedenen Ausführungsbeispielen eine Kamera aufweisen. Der Glätte-/Lockigkeitssensor kann in verschiedenen Ausführungsbeispielen eingerichtet sein, ggf. in Verbindung mit der elektronischen Schaltkreisvorrichtung, z.B. dem Prozessor, z.B. mittels eines Bildverarbeitungsprogramms, eine Glätte oder eine Lockigkeit des Haars zu ermitteln.

In verschiedenen Ausführungsbeispielen kann der Sensor einen Haarfeuchtigkeitssensor aufweisen. Der Haarfeuchtigkeitssensor kann in verschiedenen Ausführungsbeispielen eingerichtet sein, einen Wassergehalt des Haars zu ermitteln. Der Haarfeuchtigkeitssensor kann beispielsweise als Nahinfrarotspektroskop gestaltet sein, welches eingerichtet sein kann, Nahinfrarot-(NIR-)Absorptionsstrukturen von Wasser zu untersuchen und anhand dessen, ggf. unter Zuhilfenahme des Prozessors, die Haarfeuchtigkeit zu ermitteln.

In verschiedenen Ausführungsbeispielen kann der mindestens eine Sensor einen Kämmarbeit-Sensor aufweisen. Der Kämmarbeit-Sensor kann eingerichtet sein, einen Kraftaufwand zu erfassen (beispielsweise mittels Dehnmessstreifen), der bei einem Kämmen der Haare aufgewendet wird.

In verschiedenen Ausführungsbeispielen kann der mindestens eine Sensor einen Haardichtesensor aufweisen. Der Haardichtesensor kann beispielsweise eine Kamera oder einen Kameraaufsatz aufweisen, welche/r eingerichtet sein kann, direkt an eine Haarwurzelgegend gehalten zu werden, z.B. direkt auf eine Kopfhaut aufgesetzt zu werden. Eine Haardichte kann anhand des Bildes beispielsweise anhand einer Zahl von Haaren und/oder eines Abstands zwischen den Haaren, die Haardichte zu ermitteln.

In verschiedenen Ausführungsbeispielen kann der Sensor, z.B. im Fall eines Spektrometers oder einer Kamera, eingerichtet sein, mehrere Haarzustandsparameter zu ermitteln, z.B. sowohl den Haarschädigungsgrad anhand der Cysteinsäure-Absorptionsstrukturen im NIR-Spektrum als auch die Haarfeuchtigkeit anhand der Wasser-Absorptionsstrukturen im NIR-Spektrum.

In verschiedenen Ausführungsbeispielen kann der Sensor eingerichtet sein, weitere Haarzustandsparameter zu ermitteln.

In verschiedenen Ausführungsbeispielen kann die Sensorvorrichtung mindestens einen Sensor aufweisen, der eingerichtet ist zum Erfassen eines Umgebungsparameters. In verschiedenen Ausführungsbeispielen erfolgt das Erfassen eines Umgebungsparameters im Freien.

Der Umgebungssensor kann beispielsweise ein Thermometer aufweisen, welches zum Erfassen einer Umgebungstemperatur eingerichtet sein kann, einen Luftfeuchtigkeitssensor zum Erfassen einer Feuchtigkeit der Umgebungsluft, einen Windsensor, oder Ähnliches.

Alternativ kann in verschiedenen Ausführungsbeispielen ein Umgebungsparameter durch eine externe Quelle bereitgestellt werden. In verschiedenen Ausführungsbeispielen ist der Umgebungsparameter kein aktuell erfasster Parameter, sondern ein für einen vom Nutzer bestimmten Zeitpunkt prognostizierter Umgebungsparameter. Der Umgebungsparameter kann beispielsweise von Internetportalen, Internetseiten, Datenbanken oder Apps, beispielsweise Wettervorhersage-Apps, dem Nutzer bereitgestellt werden und durch diesen dem Haarbehandlungssystem, beispielsweise mittels einer Eingabevorrichtung, bereitgestellt werden. Alternativ kann der Umgebungsparameter direkt, beispielsweise über eine im Haarbehandlungssystem integrierte oder mit diesem kommunizierende Wettervorhersage-App, bereitgestellt und im Verfahren zur kosmetischen Behandeln von Haaren eines Nutzers berücksichtigt werden.

In verschiedenen Ausführungsbeispielen kann der mindestens eine Umgebungsparameter, welcher von dem mindestens einen Umgebungssensor ermittelt wird und/oder durch eine externe Quelle bereitgestellt wird, beim Steuern oder Regeln der Haarbehandlung berücksichtigt werden. Beispielsweise kann eine Temperatur während der Haarbehandlung höher sein und/oder eine Behandlungsdauer während der Haarbehandlung länger sein und/oder ein stärker wirkendes Haarbehandlungsmittel auf das Haar aufgebracht werden und/oder eine höhere Kraft auf das Haar ausgeübt werden, wenn der ermittelte mindestens eine Umgebungsparameter darauf schließen lässt, dass das behandelte Haar durch die Umgebung einer relativ starken Belastung ausgesetzt wird (z.B. durch starken Wind und/oder hohe oder niedrige Temperatur und/oder hohe Luftfeuchtigkeit (bei zu glättendem Haar) oder niedrige Luftfeuchtigkeit (bei zu lockendem Haar)). Demgegenüber kann eine Temperatur während der Haarbehandlung niedriger sein und/oder eine Behandlungsdauer während der Haarbehandlung kürzer sein und/oder ein schwächer wirkendes Haarbehandlungsmittel auf das Haar aufgebracht werden, und/oder eine geringere Kraft auf das Haar ausgeübt werden, wenn der ermittelte mindestens eine Umgebungsparameter darauf schließen lässt, dass das behandelte Haar durch die Umgebung einer relativ geringen Belastung ausgesetzt wird (z.B. durch schwachen/keinen Wind und/oder durchschnittliche Temperatur und/oder niedrige Luftfeuchtigkeit (bei zu glättendem Haar) oder hohe Luftfeuchtigkeit (bei zu lockendem Haar)).

In verschiedenen Ausführungsbeispielen kann die Haarbehandlungsvorrichtung des Haarbehandlungssystems ein heizbares Gerät aufweisen oder sein, beispielsweise ein(en) Glätteisen oder ein(en) Lockenstab oder Ähnliches. Das heißt, dass die Haarbehandlungsvorrichtung in verschiedenen Ausführungsbeispielen als eine Haarbehandlungseinheit eine Heizvorrichtung aufweisen kann. Die Heizvorrichtung kann in verschiedenen Ausführungsbeispielen je nach Ergebnis der Haaranalyse (Schädigung, Haardicke, Lockigkeit, Wassergehalt) und/oder der Umgebungsanalyse gesteuert oder geregelt werden. Beispielsweise kann die Haarbehandlungsvorrichtung oder das Haarbehandlungssystem bei intakterem, dickerem, lockigerem und/oder feuchterem Haar und/oder bei das Haar stärker beanspruchenden Umgebungsbedingungen (z.B. stärkerer Wind, höhere Temperatur), so gesteuert oder geregelt werden, dass das Haar mit einer höheren Temperatur behandelt wird, als wenn das Haar geschädigter, dünner, glatter und/oder trockener ist.

In verschiedenen Ausführungsbeispielen kann das Glätteisen oder der Lockenstab, als eine Haarbehandlungseinheit, eine Vorrichtung aufweisen, die eingerichtet ist, an dem Haar mechanische Arbeit zu verrichten, beispielsweise Druck oder Zug auf das Haar auszuüben. Dafür kann die Vorrichtung mit einem Motor ausgerüstet sein. Mittels des (z.B. elektronisch ansteuerbaren) Motors kann der Druck oder Zug steuer- oder regelbar sein.

In verschiedenen Ausführungsbeispielen kann das Glätteisen oder der Lockenstab anstelle des Motors oder zusätzlich dazu mindestens einen Sensor aufweisen, der eingerichtet ist, eine Kraft zu messen, welche auf das Haar ausgeübt wird, also beispielsweise einen Zugkraftsensor oder einen Drucksensor. Diese Art von Sensoren, welche während der Haarbehandlung zum Einsatz kommen, werden hierin auch als Behandlungssensoren bezeichnet.

In einem Fall, dass der Motor vorliegt, kann die auf das Haar ausgeübte Kraft anhand der vom Zugkraft- oder Drucksensor ermittelten Werte geregelt werden, z.B. derart, dass eine vorbestimmte Kraft erreicht wird.

In einem Fall, dass die Kraft auf das Haar von Hand ausgeübt wird, kann diese vom Zugkraft- oder Drucksensor ermittelt werden, und dem Nutzer, oder der Person, die die Kraft ausübt, kann eine Rückmeldung gegeben werden, z.B. visuell oder akustisch, z.B. derart, dass die Person die von ihr ausgeübte Kraft so anpassen kann, dass eine vorbestimmte Kraft erreicht wird.

In verschiedenen Ausführungsbeispielen kann die Sensorvorrichtung, als Teil der Haarbehandlungsvorrichtung oder als separate Vorrichtung, eine Mehrzahl von (z.B. gleichartigen) Sensoren aufweisen, welche z.B. derart angeordnet sein können, dass der Nutzer sofort eine Rückmeldung darüber erhalten kann, ob eine weitere Behandlung (auch als "Post-Treatment" bezeichnet) notwendig ist.

In verschiedenen Ausführungsbeispielen kann die Haarbehandlungsvorrichtung an zwei Positionen entlang einer Bewegungsrichtung des Haars Sensoren aufweisen, wobei ein Sensor einen Haarzustandsparameter vor der Haarbehandlung (z.B. der Temperaturbehandlung und/oder der Behandlung mit dem Haarbehandlungsmittel) und ein Sensor einen (z.B. denselben) Haarzustandsparameter nach der Haarbehandlung misst. Diese Sensoren können somit als Behandlungssensoren betrachtet werden.

In verschiedenen Ausführungsbeispielen kann die Haarbehandlungsvorrichtung eine Aufbringvorrichtung zum Aufbringen des Haarbehandlungsmittels auf das Haar aufweisen. Die Aufbringvorrichtung kann beispielsweise einen Vorratsbehälter, auch als Tank oder Kartusche bezeichnet, und eine Dosiervorrichtung, z.B. mindestens ein (z.B. elektronisch ansteuerbares) Ventil und/oder eine (z.B. elektronisch ansteuerbare) Pumpvorrichtung aufweisen. Die Dosiervorrichtung kann als Teil des Tanks gebildet sein, und/oder als Teil eines Körpers des Glätteisens/des Lockenstabs, auch als Vorrichtungskörper bezeichnet.

Die Kartusche kann in verschiedenen Ausführungsbeispielen wiederbefüllbar sein.

In dem Vorratsbehälter kann sich das Haar-Behandlungsmittel, z.B. ein Stylingmittel, befinden. Je nach Ergebnis der Haaranalyse (z.B. Schädigung, Haardicke, Lockigkeit, Wassergehalt) und/oder der Umgebungsanalyse (z.B. Wind, Temperatur, Luftfeuchtigkeit) kann das Haarbehandlungsmittel, das z.B. eine (z.B. chemische) Zusammensetzung aufweisen kann, auf das Haar aufgebracht werden, indem die Dosiervorrichtung mittels des Aktuators betätigt wird.

In verschiedenen Ausführungsbeispielen können unterschiedliche Volumina/Mengen je nach Position (z.B. Ansatz, Mitte, Spitzen) aufgebracht werden oder auf das Haar aufbringbar sein.

In verschiedenen Ausführungsbeispielen können mittels des Haarbehandlungssystems zwei oder mehr Mittel in unterschiedlichen Mischungsverhältnissen je nach Position (z.B. Ansatz, Mitte, Spitzen) oder an unterschiedlichen Positionen auf das Haar aufgebracht werden oder auf das Haar aufbringbar sein.

In verschiedenen Ausführungsbeispielen kann ein Nutzer bereits während einer Durchführung einer Haarbehandlung Informationen über einen Verlauf der Anwendung erhalten. Ein Haarbehandlungsergebnis kann noch während der Durchführung der Haarbehandlung optimiert werden, so dass Frustrationen vermieden werden können.

In verschiedenen Ausführungsbeispielen kann ein Haarbehandlungssystem mit einer Haarbehandlungsvorrichtung in Form eines Glätteisens oder eines Lockenstabs bereitgestellt werden, bei welchem der mindestens eine Sensor Teil einer separaten kommunikationsfähigen (d.h. eine Datenaustauschvorrichtung aufweisenden) Sensorvorrichtung ist (beispielsweise ein Smartphone, das eine Kamera aufweist, ein Spektrometer mit der Datenaustauschvorrichtung, ein so genannter "akustischer Kamm", welcher eingerichtet sein kann, beim Kämmen entstehende Geräusche zu erfassen und zu übermitteln.

Eine elektronische Schaltkreisvorrichtung, die einen Prozessor aufweisen kann, und ein erster Aktuator, der eine Aufbringvorrichtung für ein Haarstylingmittel aufweisen kann, können in das Glätteisen oder den Lockenstab integriert sein.

In verschiedenen Ausführungsbeispielen kann das Glätteisen oder der Lockenstab als einen weiteren Aktuator eine Temperatursteuerung oder eine Temperaturregelung aufweisen, welche eine Temperatur beheizbarer Flächen des Glätteisens oder Lockenstabs steuert oder regelt. Alternativ oder zusätzlich kann in verschiedenen Ausführungsbeispielen das Glätteisen oder der Lockenstab einen oder mehrere weitere Aktuatoren aufweisen, z.B. zum Steuern oder Regeln eines Drucks, welcher auf das Haar mittels der Haarbehandlungsvorrichtung ausgeübt wird.

In verschiedenen Ausführungsbeispielen kann das Glätteisen oder der Lockenstab eine Datenaustauschvorrichtung aufweisen, beispielsweise zum Empfangen von mittels des mindestens einen Sensors erfassten Messwerten und/oder zum Empfangen von Haarbehandlungsparametern, z.B. Steuerungsanweisungen.

In verschiedenen Ausführungsbeispielen kann der mindestens eine Sensor, statt Teil der separaten kommunikationsfähigen Sensorvorrichtung zu sein oder (z.B. im Fall mehrerer Sensoren) ergänzend dazu, in das Glätteisen oder den Lockenstab integriert sein.

In verschiedenen Ausführungsbeispielen kann das Haarbehandlungssystem zusätzlich zu der elektronischen Schaltkreisvorrichtung eine Datenverarbeitungsvorrichtung aufweisen oder eingerichtet sein, mit der Datenverarbeitungsvorrichtung Daten auszutauschen. Die Datenverarbeitungsvorrichtung kann kommunikationsfähig sein, d.h. eine Datenaustauschvorrichtung aufweisen, z.B. Teil eines Smartphones, eines Tablets, eines Laptops, eines Smart Mirrors oder Ähnliches sein, auf dem beispielsweise eine App installiert sein kann, oder beispielsweise eine Cloud oder ähnliches).

In verschiedenen Ausführungsbeispielen kann das Glätteisen oder der Lockenstab eine Datenaustauschvorrichtung aufweisen, beispielsweise zum Empfangen von mittels der Sensorvorrichtung ermittelten Haarzustands- und/oder Umgebungsparameter oder von daraus ermittelten Behandlungsparametern, und/oder zum Empfangen von mittels der Datenverarbeitungsvorrichtung ermittelten Empfehlungen und/oder Steuerungsanweisungen.

Mittels eines Softwareprogramms, z.B. einer (Smartphone-)App, können gezielt Informationen, z.B. Steuerungs- oder Regelungsanweisungen, an die Haarbehandlungsvorrichtung (z.B. ein "smartes" Glätteisen oder Lockenstab) übertragen werden, welche eingerichtet sein kann, anhand der Steuerungs- oder Regelungsanweisungen einen Haarbehandlungsparameter zu steuern oder regeln, z.B. ein Haarbehandlungsmittel zu dosieren, eine Temperatur einzustellen, mit der die Haare ohne weitere Schädigung behandelt werden können, usw. Ein Datenaustausch kann hierbei kabellos erfolgen, z.B. über Bluetooth oder eine Near Field Communication Technologie (NFC-Technologie).

In einer beispielhaften Ausführungsform kann die mindestens eine Pumpe mittels eines Smartphones o.ä., z.B. mittels einer App, gesteuert oder geregelt werden. Das Smartphone kann die Datenverarbeitungsvorrichtung bilden, welche Sensordaten von der Sensorvorrichtung empfängt und/oder selbst mindestens einen Zustandssensor bereitstellt, z.B. die Smartphonekamera (ggf. im Zusammenhang mit einer geeigneten App zum Ermitteln mindestens eines Haarzustandsparameters, z.B. einer Lockigkeit des Haars, einer Haarfarbe o.ä.). Anhand des mindestens einen empfangenen und/oder ermittelten Haarzustandsparameters kann (z.B. mittels einer geeigneten Software, z.B. einer App) mittels des Smartphones ein Behandlungsparameter ermittelt werden. Beispielsweise kann mittels des Smartphones eine Flussrate des Haarbehandlungsmittels ermittelt und, mittels Übertragens eines Dosierbefehls vom Smartphone an die Haarbehandlungsvorrichtung, in dem Glätteisen/dem Lockenstab eingestellt werden.

In verschiedenen Ausführungsbeispielen kann ein Haarbehandlungssystem bereitgestellt werden, welches eine Haarbehandlungsvorrichtung (z.B. ein Glätteisen, einen Lockenstab o.ä.) aufweisen kann, und ferner eine Datenverbindung (für die Möglichkeit des Datenaustauschs ist auch der Begriff Konnektivität oder Connectivity gebräuchlich) aufweisen kann zwischen einer externen App und der Haarbehandlungsvorrichtung (z.B. dem Lockenstab oder dem Glätteisen). In verschiedenen Ausführungsbeispielen können Pflege/Styling-Parameter bereitgestellt, z.B. vorgegeben, werden (z.B. eine maximale Temperatur), wobei der bereitgestellte Parameter bezogen sein kann auf einen Schädigungsgrad des Haares, d.h. je nach Schädigungsgrad des Haares kann der Parameter einen anderen Wert aufweisen.

In verschiedenen Ausführungsbeispielen kann die Eingabevorrichtung eingerichtet sein, mindestens eine Eingabe durch den Nutzer aufzunehmen und der elektronischen Schaltkreisvorrichtung und/oder einer externen Datenverarbeitungsvorrichtung bereitzustellen. Vom Nutzer einzugebende Parameter oder Informationen können beispielsweise Alter und/oder Geschlecht, eine Wunschfrisur, eine aktuelle Haarlänge, eine Haarlockigkeit oder ähnliches aufweisen.

Eine oder mehrere der Informationen können in verschiedenen Ausführungsbeispielen als Teil eines Nutzerprofils abgespeichert werden, z.B. in der elektronischen Schaltkreisvorrichtung und/oder in der externen Datenverarbeitungsvorrichtung und somit bei einer späteren Verwendung wieder abrufbar sein.

In verschiedenen Ausführungsbeispielen können für die Eingabe beispielsweise Vergleichsbilder bereitgestellt werden, aus welchen der Nutzer dasjenige auswählen kann (z.B. mittels Antippens/Anklickens o.ä.), welches seinem gegenwärtigen und/oder angestrebten Zustand (auch als Wunschzustand oder Zielzustand bezeichnet, im Fall einer Frisur auch als Wunschfrisur) am Nächsten kommt. Anstelle von mittels des Systems bereitgestellten Vergleichsbildern oder ergänzend dazu kann mittels des Nutzers dem System ein Bild bereitgestellt werden, beispielsweise ein digitales Bild, auf welchem eine Frisur des Nutzers abgebildet ist, und/oder ein digitales Bild, auf welchem eine Wunschfrisur des Nutzers abgebildet ist, z.B. ein Prominenter, ein Freund/eine Freundin des Nutzers mit der Wunschfrisur oder ein Frisurenmodell aus einem "Look Book".

Die vom Nutzer bereitgestellte Information, z.B. hinsichtlich seines Wunschzustands, kann in verschiedenen Ausführungsbeispielen mittels der elektronischen Schaltkreisvorrichtung und/oder der externen Datenverarbeitungsvorrichtung berücksichtigt werden beim Ermitteln der Haarbehandlung, z.B. des mindestens einen Haarbehandlungsparameters.

In verschiedenen Ausführungsbeispielen kann unter Berücksichtigung der ermittelten Haarzustands- und/oder Umgebungsparameter und der vom Nutzer bereitgestellten Information eine Schritt-für-Schritt-Anleitung bereitgestellt werden, wie der Wunschzustand, z.B. die Wunschfrisur, zu erreichen ist. Die Anleitung kann in verschiedenen Ausführungsbeispielen mittels der Ausgabevorrichtung bereitgestellt werden, beispielsweise als Bilderfolge oder Video und/oder als Sprachanweisung. Die Anleitung kann beispielsweise derart bereitgestellt werden, dass der Nutzer eine benötigte Stelle in der Abfolge selbst anwählt, und/oder derart, dass ein Abschließen eines der Schritte der Anleitung automatisch mittels des Zustands- und/oder Behandlungssensors erfasst wird und/oder vom Nutzer angezeigt wird, und daraufhin der nachfolgende Schritt der Anleitung bereitgestellt wird.

Das zum Steuern oder Regeln der Haarbehandlung gesteuerte oder geregelte Element kann eine Dosiervorrichtung für das Haarbehandlungsmittel und/oder eine Heizvorrichtung zum Erwärmen des Haars und/oder einen Motor zum Ausüben einer Kraft auf das Haar aufweisen. Das gesteuerte oder geregelte Element kann in verschiedenen Ausführungsbeispielen mittels einer drahtlosen Übertragungsvorrichtung gesteuert werden, beispielsweise indem die Aufbringvorrichtung Steuerbefehle mittels der drahtlosen Übertragungsvorrichtung empfängt. Die drahtlose Übertragungsvorrichtung kann beispielsweise Teil der elektronischen Schaltkreisvorrichtung sein. Die drahtlose Übertragungsvorrichtung kann in verschiedenen Ausführungsbeispielen einen Chip oder Tag aufweisen, der die drahtlose Datenübertragung, z.B. mittels Bluetooth, WLAN, ZigBee, NFC, Wibree, Thread, WiMAX oder ähnlichem ermöglicht.

In verschiedenen Ausführungsbeispielen kann vor einem Bereitstellen einer Empfehlung an den Nutzer ein Datenabgleich vorgenommen werden zwischen dem smarten Endgerät und/oder dem smarten Stylinggerät und Daten, z.B. Referenzdaten, welche beispielsweise in einer Cloud hinterlegt sein können. Die Daten können in verschiedenen Ausführungsbeispielen Daten von anderen Nutzern aufweisen, welche beispielsweise denselben mindestens einen Haarzustandsparameter aufweisen, und beispielsweise entsprechende abgeleitete Empfehlungen/Maßnahmen.

In verschiedenen Ausführungsbeispielen kann die ermittelte Empfehlung genutzt werden für eine Herstellung eines optimalen/personalisiertem Stylingprodukts, z.B. als ein Auftrag für eine Herstellung eines optimalen/personalisiertem Stylingprodukts.

In verschiedenen Ausführungsbeispielen kann ein herkömmliches Haarbehandlungsmittel verwendet werden.

In verschiedenen Ausführungsbeispielen kann eine Nutzereingabe an einer Aufbringvorrichtung zur optimierten Abgabe des Stylingprodukts genutzt werden. Der Nutzer kann mithilfe einer digitalen Anzeige und eines Touchscreens die gewünschte Menge an Produkt auswählen. Hierzu kann eine gespeicherte Empfehlung der Experten bereits im Gerät einprogrammiert sein, beispielsweise in einer Datenbank abgelegt (d.h. gespeichert) sein. Mittels einer vorherigen individuellen Eingabe der Haarlänge des Nutzers kann so eine (Mengen- und) Produktempfehlung abgegeben werden. Diese kann vom Nutzer entweder bestätigt, erweitert oder verringert werden, wenn dieser selbst schon weiß, dass er tendenziell mehr/weniger Produkt anwendet, als auf Verpackungen normalerweise angegeben.

In verschiedenen Ausführungsbeispielen wird ein Verfahren zum kosmetischen Behandeln von Haaren bereitgestellt, welches eine richtige, d.h. zum Haarschädigungsgrad passende, Produktauswahl ermöglicht. Beispielsweise kann bei der Haarbehandlung ein exakt passendes Produkt für ein Haarstyling angesichts seines Haarschädigungsgrads verwendet werden.

Hierin kann Bezug genommen werden auf "die Sensoren", beispielsweise hinsichtlich einer Datenübertragung zwischen den Sensoren und einer Datenverarbeitungsvorrichtung, einer Anordnung der Sensoren, usw. Dies ist so zu verstehen, dass die Sensoren eine Gesamtheit von in der Sensorvorrichtung oder der Haarbehandlungsvorrichtung angeordneten Sensoren aufweisen kann, z.B. eine Gesamtheit von Kamera/s, Temperatursensor/en, Mikrofon/en, usw., oder, sofern dies aus dem Kontext hervorgeht, einen Teil der genannten Sensoren.

In verschiedenen Ausführungsbeispielen kann das Haarbehandlungssystem eine elektronische Vorrichtung aufweisen, z.B. eine mobile elektronische Vorrichtung (auch als Mobile Device bezeichnet), z.B. ein Smartphone oder ein Tablet, oder z.B. eine sonstige Datenverarbeitungsvorrichtung (z.B. einen PC). In verschiedenen Ausführungsbeispielen kann das Haarbehandlungssystem ferner eine (ggf. weitere) externe Datenverarbeitungsvorrichtung, z.B. eine Cloud, nutzen für eine Signalauswertung, z.B. als eine Erweiterung der Signalauswertung. Dafür können in verschiedenen Ausführungsbeispielen die mittels der Sensoren erfassten Signale mit in einer Datenbank hinterlegten Signalen (auch als Vergleichssignale, Vergleichsdaten, Referenzsignale oder Referenzdaten bezeichnet) verglichen werden. In verschiedenen Ausführungsbeispielen kann anhand dessen der Haarschädigungsgrad oder ein sonstiger Haarzustandsparameter eingeordnet werden, beispielsweise indem den Vergleichssignalen Haarschädigungsgrade zugeordnet sind, und der Haarschädigungsgrad oder der sonstige Haarzustandsparameter des dem gemessenen Signals ähnlichsten Vergleichssignals den gemessenen Haaren zugeordnet wird.

Die Referenzdaten, die z.B. als eine Datenbank bereitgestellt sein können, können in verschiedenen Ausführungsbeispielen empirisch (z.B. im Labor) gewonnen sein für Haare, deren Haarschädigungsgrad bekannt sein kann. In verschiedenen Ausführungsbeispielen können ferner weitere Informationen über die Haare vorliegen, die als Grundlage für die Referenzspektren dienen, z.B. "viermal gebleichtes Haar - hoher Schädigungsgrad" oder "unbehandeltes Haar - keine Schädigung", und/oder für die Haare, die für das Ermitteln der Referenzspektren genutzt werden, kann eine Entwicklung eines Haarzustands, z.B. des Haarschädigungsgrads, bereitgestellt sein, z.B. mehrere Referenzspektren, von denen jedes nach einer anderen Haarbehandlungsstufe aufgenommen wurde, wobei die Haarbehandlung eine pflegende Haarbehandlung und/oder eine schädigende Haarbehandlung aufweisen kann. Ein Mittel (z.B. Produkt und/oder Inhaltsstoff), welches bei einer Behandlung genutzt wurde, kann außerdem mittels der Datenbank erfasst worden sein.

In verschiedenen Ausführungsbeispielen kann einem Nutzer auch die (z.B. der Datenbank entnommene) Zusatzinformation bereitgestellt werden, beispielsweise welchem Behandlungsgrad sein Haarzustand entspricht, und/oder wie sich sein Haarzustand voraussichtlich entwickeln wird, wenn er eine bestimmte Behandlung vornimmt, z.B. ein bestimmtes Mittel anwendet.

In verschiedenen Ausführungsbeispielen, z.B. bei Verwendung einer Cloud, kann die Datenbank alternativ zu einem Erzeugen im Labor mittels Nutzerdaten erzeugt werden (und beispielsweise fortlaufend ergänzt werden). In verschiedenen Ausführungsbeispielen kann die im Labor erzeugte Datenbank mittels Nutzerdaten, welche mittels der Cloud bereitgestellt werden können, ergänzt werden.

In verschiedenen Ausführungsbeispielen können die aufgezeichneten Daten, wie hierin an anderer Stelle beschrieben, mittels Softwarealgorithmen analysiert werden, um einen Haarzustandsparameter, z.B. einen Haarschädigungsgrad, und/oder einen Umgebungsparameter zu ermitteln.

In verschiedenen Ausführungsbeispielen, beispielsweise wenn die Referenzdaten mittels der Cloud bereitgestellt werden, können diese einem Nutzer jederzeit zur Verfügung stehen, um als Referenzdaten für einen Vergleich genutzt zu werden.

In verschiedenen Ausführungsbeispielen können die mittels der Haarbehandlungsvorrichtung oder mittels des Haarbehandlungssystems erfassten Daten gespeichert werden, beispielsweise in einem in der Haarbehandlungsvorrichtung integrierten Speicher und/oder in der externen Datenverarbeitungsvorrichtung, z.B. der Cloud. Die gespeicherten Daten können so abgespeichert werden, dass zumindest dem Nutzer ermöglicht ist, diese Daten als seine Daten zu erkennen. Damit kann ein Vergleichen von Haarinformationen, die beispielsweise zu verschiedenen Zeitpunkten gewonnen wurden (z.B. vor und nach einer Behandlung) miteinander ermöglicht sein.

In verschiedenen Ausführungsbeispielen kann das Haarbehandlungssystem eine Verbindung zum Übertragen (Senden und/oder Empfangen) von Daten aufweisen, beispielsweise zwischen einem Smartphone/Tablet, welches Teil des Haarbehandlungssystems sein kann, und einer Cloud, und/oder zwischen der Haarbehandlungsvorrichtung und dem Smartphone/Tablet, und/oder zwischen der Haarbehandlungsvorrichtung und der Cloud, und/oder zwischen der Sensorvorrichtung und der Haarbehandlungsvorrichtung und/oder zwischen der Sensorvorrichtung und dem Smartphone/Tablet, und/oder zwischen der Sensorvorrichtung und der Cloud.

In verschiedenen Ausführungsbeispielen kann die Analyse durch die Haarbehandlungsvorrichtung, beispielsweise mittels der Schaltkreisvorrichtung, erfolgen, und ein Analyseergebnis kann zum Bereitstellen des Analyseergebnisses an eine Anzeigevorrichtung übertragen werden, beispielsweise an ein Display, einen Lautsprecher, ein Smartphone oder ähnliches.

In verschiedenen Ausführungsbeispielen können die Daten übertragen werden auf/an eine Datenverarbeitungsvorrichtung, z.B. auf ein Smartphone mit App, an eine Cloud, usw. Nach der Datenübertragung auf die Datenverarbeitungsvorrichtung kann mittels dieser die Untersuchung der Daten ausgeführt werden, beispielsweise zum Ermitteln eines Steuerungs- oder Regelungsparameters. Die Datenverarbeitungsvorrichtung kann in verschiedenen Ausführungsbeispielen Teil des Haarbehandlungssystems sein, z.B. in einem Fall, dass die Datenverarbeitungsvorrichtung, z.B. das Smartphone o.ä., Teil der Sensorvorrichtung und/oder der Ein- und/oder Ausgabevorrichtung oder ein eigenständiger Teil des Haarbehandlungssystems ist (auch als integrierte Datenverarbeitungsvorrichtung bezeichnet).

In verschiedenen Ausführungsbeispielen kann die Datenverarbeitungsvorrichtung eine externe Datenverarbeitungsvorrichtung sein, beispielsweise die Cloud.

In verschiedenen Ausführungsbeispielen kann der Steuerungs- oder Regelungsparameter oder die Empfehlung direkt mittels des Haarbehandlungssystems, z.B. der Haarbehandlungsvorrichtung und/oder der Sensorvorrichtung und/oder der Eingabe- oder Ausgabevorrichtung und/oder der sonstigen integrierten Datenverarbeitungsvorrichtung, ermittelt sein oder werden, d.h. die elektronische Schaltkreisvorrichtung und/oder die integrierte Datenverarbeitungsvorrichtung kann eingerichtet sein, den Steuerungs-/Regelungsparameter und ggf. weitere Informationen selbst (auch als direkt bezeichnet) zu ermitteln. Beispielsweise kann die elektronische Schaltkreisvorrichtung eine Datenverarbeitungsvorrichtung sein oder aufweisen, z.B. kann sie mit einem Speicher und einem Prozessor, z.B. einem Mikroprozessor, ausgerüstet sein, welche eingerichtet sein können, z.B. mittels einer Programmierung, die Sensordaten zu empfangen und die Haarbehandlungsvorrichtung zu steuern oder zu regeln, und ggf. um dem Nutzer Informationen bereitzustellen. Beispielsweise können die Sensordaten wie oben beschrieben mit einer Datenbank verglichen werden.

In verschiedenen Ausführungsbeispielen kann die elektronische Schaltkreisvorrichtung eingerichtet sein, den Steuerungs- oder Regelungsparameter oder die Empfehlung, z.B. Produkt- oder Behandlungsempfehlung, indirekt zu ermitteln. Beispielsweise kann die elektronische Schaltkreisvorrichtung (z.B. zusätzlich zu einem Speicher und einem Prozessor, z.B. einem Mikroprozessor) mit einer Datenübertragungsvorrichtung ausgerüstet sein, welche eingerichtet sein kann, die von der elektronische Schaltkreisvorrichtung empfangenen Sensordaten an eine externe Datenverarbeitungsvorrichtung, z.B. an einen Computer, z.B. eine Cloud, zu übermitteln, mittels derer, beispielsweise wie oben für das Ermitteln der Steuerungs- oder Regelungsparameter oder der Empfehlung mittels der elektronischen Schaltkreisvorrichtung beschrieben, der Steuerungs-oder Regelungsparameter oder die Empfehlung ermittelt werden kann, um den Steuerungs- oder Regelungsparameter oder die Empfehlung bereitzustellen, beispielsweise mittels Übertragens an die Haarbehandlungsvorrichtung und/oder an die Anzeigevorrichtung und/oder mittels Übertragens der Empfehlung zurück an die elektronische Schaltkreisvorrichtung (z.B. mittels der Datenübertragungsvorrichtung). In verschiedenen Ausführungsbeispielen kann die Datenübertragung mehrstufig erfolgen, beispielsweise indem die Sensordaten von der Schaltkreisvorrichtung zunächst an die Anzeigevorrichtung (z.B. ein Smartphone, ein Tablet o.ä.) übermittelt werden, und die Anzeigevorrichtung die Sensordaten an die externe Datenverarbeitungsvorrichtung (z.B. die Cloud) überträgt.

Erfindungsgemäß wird ein Haarbehandlungssystem gemäß Anspruch 1 bereitgestellt. Das Haarbehandlungssystem weist eine Sensorvorrichtung mit mindestens einem Zustandssensor zum Erfassen Haarschädigungsgrads auf und eine elektrische Haarbehandlungsvorrichtung. Die Haarbehandlungsvorrichtung weist eine Aufbringvorrichtung zum Aufbringen eines Haarbehandlungsmittels auf das Haar, eine Haarbehandlungseinheit und eine elektronische Schaltkreisvorrichtung auf, wobei die elektronische Schaltkreisvorrichtung mit der Sensorvorrichtung gekoppelt ist zum Empfangen des erfassten Haarschädigungsgrad, und wobei die elektronische Schaltkreisvorrichtung eingerichtet ist, basierend auf dem empfangenen Haarschädigungsgrad die Haarbehandlung zu steuern, wobei die Haarbehandlung ein Aufbringen des Haar-Behandlungsmittels mittels der Aufbringvorrichtung und eine mechanische und/oder thermische Behandlung mittels der Haarbehandlungseinheit aufweist, wobei die Sensorvorrichtung mindestens einen Sensor aufweist, der eingerichtet ist zum Erfassen eines Umgebungsparameters.

In verschiedenen Ausführungsbeispielen kann die Haarbehandlungsvorrichtung ein Glätteisen oder einen Lockenstab aufweisen.

In verschiedenen Ausführungsbeispielen kann der mindestens eine Zustandssensor mindestens einen aufweisen aus einer Gruppe von Zustandssensoren, die Gruppe aufweisend: eine Kamera zum Aufnehmen eines Bildes mittels sichtbaren Lichts, UV-Lichts und/oder Nahinfrarotlichts zum Ermitteln eines Haarschädigungsgrads, einer Lockigkeit, einer Haarfarbe, einer Haardicke und/oder einer Haardichte, ein Mikroskop zum Ermitteln eines Haarschädigungsgrads und/oder einer Haardicke, ein Spektrometer zum Aufnehmen eines Spektrums von sichtbarem Licht, UV-Licht und/oder Nahinfrarotlicht zum Ermitteln eines Haarschädigungsgrads, eines Haaraminosäuregehalts und/oder eines Haarfeuchtigkeitsgehalts, einen akustischen Sensor zum Ermitteln eines Haarschädigungsgrads, einen Temperatursensor zum Ermitteln einer Umgebungstemperatur, einen Luftfeuchtigkeitssensor und einen Windsensor.

In verschiedenen Ausführungsbeispielen können die Sensorvorrichtung und die Haarbehandlungsvorrichtung separate Vorrichtungen sein.

In verschiedenen Ausführungsbeispielen können zumindest ein Teil der Sensorvorrichtung und die Haarbehandlungsvorrichtung eine integrierte Einheit bilden.

In verschiedenen Ausführungsbeispielen kann die elektronische Schaltkreisvorrichtung eine kabellose Datenaustauschvorrichtung aufweisen.

In verschiedenen Ausführungsbeispielen kann die Haarbehandlungsvorrichtung mindestens ein gesteuertes oder geregeltes Element zum Steuern oder Regeln der Haarbehandlung aufweisen.

In verschiedenen Ausführungsbeispielen kann das gesteuerte oder geregelte Element eine Dosiervorrichtung für das Haarbehandlungsmittel und/oder, als die Haarbehandlungseinheit, eine Heizvorrichtung und/oder einen Motor zum Ausüben einer Kraft auf das Haar aufweisen.

In verschiedenen Ausführungsbeispielen kann die Haarbehandlungsvorrichtung ferner einen Behandlungssensor aufweisen.

In verschiedenen Ausführungsbeispielen kann die Steuerung der Haarbehandlung ferner auf einem vom Nutzer vorab ausgewählten Zielzustand basieren.

In verschiedenen Ausführungsbeispielen kann das Haarbehandlungssystem ferner eine Eingabevorrichtung und/oder eine Ausgabevorrichtung aufweisen.

In verschiedenen Ausführungsbeispielen kann die Eingabevorrichtung einen berührungsempfindlichen Bildschirm und/oder eine Tastatur und/oder eine Maus und/oder ein Mikrofon aufweisen, und/oder die Ausgabevorrichtung kann einen Bildschirm und/oder einen Lautsprecher aufweisen.

In verschiedenen Ausführungsbeispielen kann das System ferner eine Datenverarbeitungsvorrichtung aufweisen oder mit der Datenverarbeitungsvorrichtung verbindbar sein.

In verschiedenen Ausführungsbeispielen kann die Datenverarbeitungsvorrichtung eine externe Datenverarbeitungsvorrichtung sein.

In verschiedenen Ausführungsbeispielen kann die externe Datenverarbeitungsvorrichtung eine Cloud aufweisen oder sein.

Erfindungsgemäß wird ein Verfahren zum kosmetischen Behandeln von Haaren eines Nutzers gemäß Anspruch 13 bereitgestellt. Das Verfahren weist auf: vor einem Behandeln oder während des Behandelns der Haare mittels eines Haarbehandlungssystems gemäß Anspruch 1 Erfassen eines Haarschädigungsgrads mittels des mindestens einen Zustandssensors, und Steuern oder Regeln einer Haarbehandlung basierend auf dem empfangenen erfassten Haarschädigungsgrads, wobei die Haarbehandlung ein Aufbringen des Haar-Behandlungsmittels und eine mechanische und/oder thermische Behandlung aufweist.

In verschiedenen Ausführungsbeispielen kann das Verfahren ferner während der Haarbehandlung ein Erfassen eines Haarbehandlungsparameters mittels eines Haarbehandlungssensors aufweisen.

In verschiedenen Ausführungsbeispielen kann das Verfahren ferner ein Übermitteln des erfassten Haarschädigungsgrads an die elektronische Schaltkreisvorrichtung aufweisen. Ausführungsbeispiele der Erfindung sind in den Figuren dargestellt und werden im Folgenden näher erläutert.

Es zeigen
Figur 1A, 1B und 1C jeweils eine schematische Darstellung eines Haarbehandlungssystems gemäß verschiedenen Ausführungsbeispielen;
Figur 2A bis Figur 2F jeweils eine schematische Darstellung eines Haarbehandlungssystems gemäß verschiedenen Ausführungsbeispielen;
Figur 3A und 3B jeweils eine schematische Darstellung einer Anwendung eines Haarbehandlungssystems gemäß verschiedenen Ausführungsbeispielen; und
Figur 4 ein Flussdiagramm eines Verfahrens zum kosmetischen Behandeln von Haaren gemäß verschiedenen Ausführungsbeispielen.

In der folgenden ausführlichen Beschreibung wird auf die beigefügten Zeichnungen Bezug genommen, die Teil der vorliegenden Anmeldung bilden und in denen zur Veranschaulichung spezifische Ausführungsformen gezeigt sind, in denen die Erfindung ausgeübt werden kann. In dieser Hinsicht wird Richtungsterminologie wie etwa "oben", "unten", "vorne", "hinten", "vorderes", "hinteres", usw. mit Bezug auf die Orientierung der beschriebenen Figur(en) verwendet. Da Komponenten von Ausführungsformen in einer Anzahl verschiedener Orientierungen positioniert werden können, dient die Richtungsterminologie zur Veranschaulichung und ist auf keinerlei Weise einschränkend. Es versteht sich, dass andere Ausführungsformen benutzt und strukturelle oder logische Änderungen vorgenommen werden können, ohne von dem Schutzumfang der vorliegenden Erfindung abzuweichen. Es versteht sich, dass die Merkmale der hierin beschriebenen verschiedenen beispielhaften Ausführungsformen miteinander kombiniert werden können, sofern nicht spezifisch anders angegeben. Die folgende ausführliche Beschreibung ist deshalb nicht in einschränkendem Sinne aufzufassen, und der Schutzumfang der vorliegenden Erfindung wird durch die angefügten Ansprüche definiert.

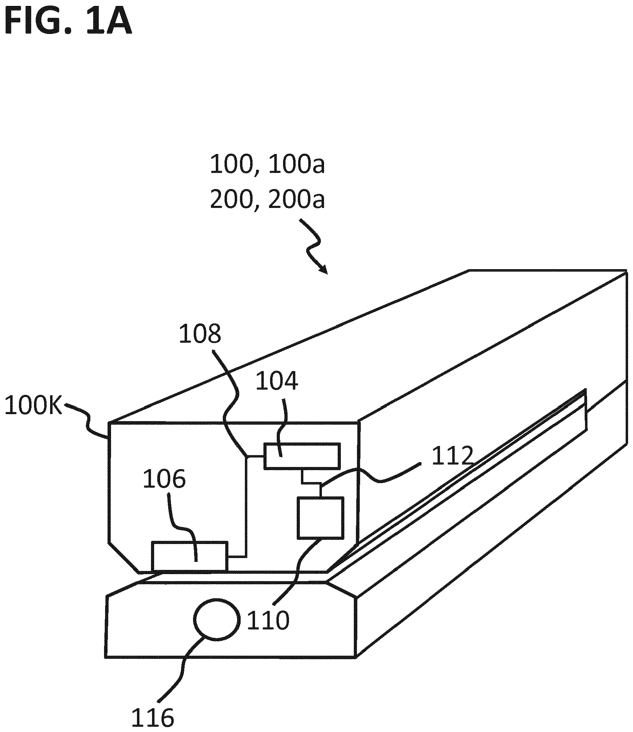

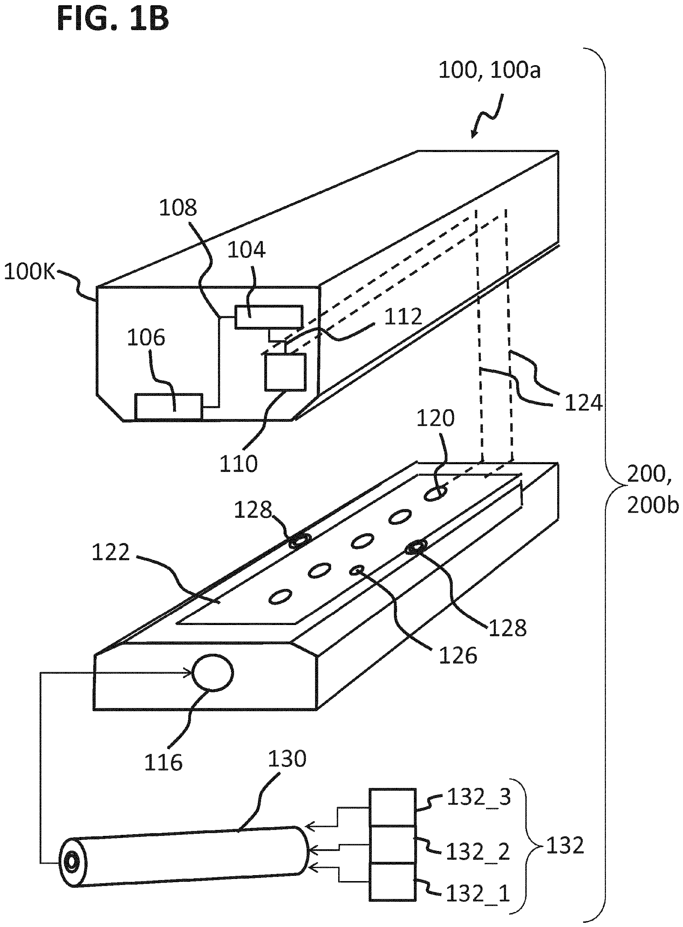

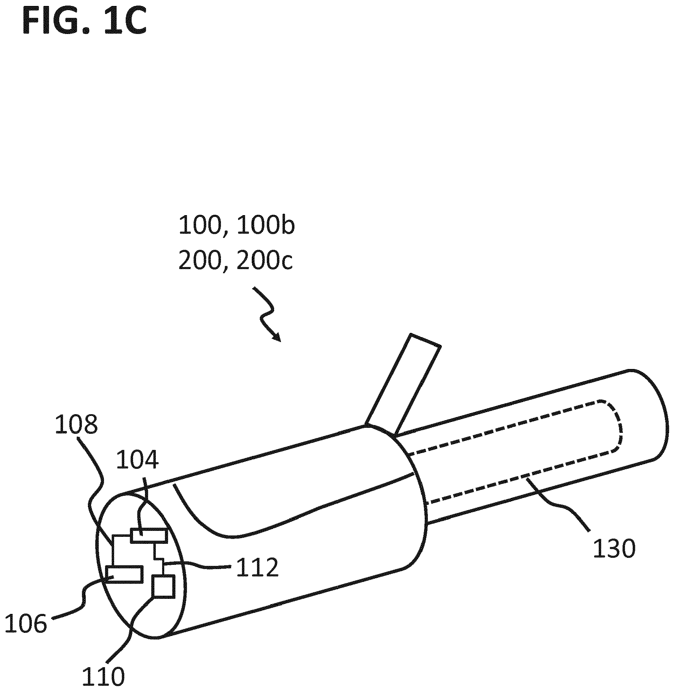

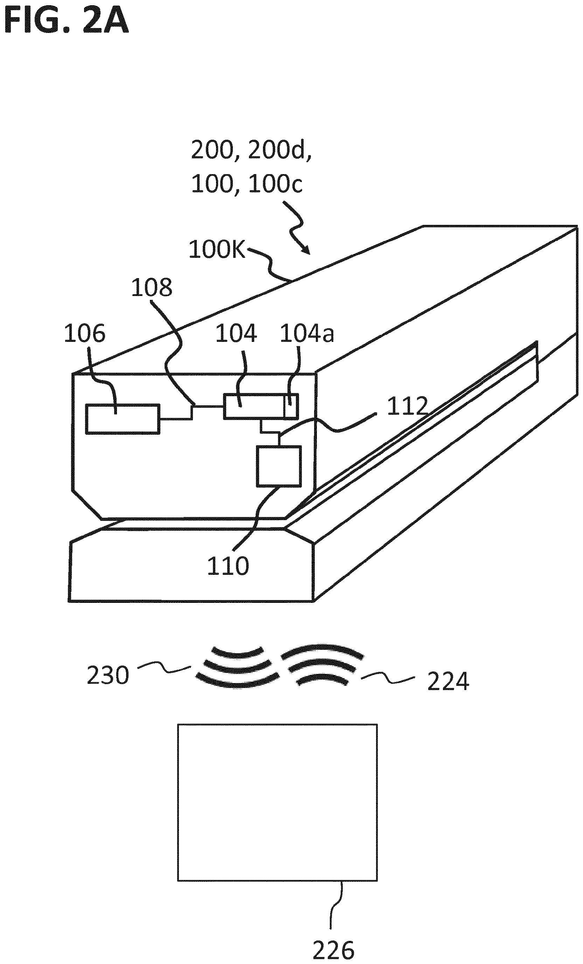

Sofern nicht ohnehin "Steuern oder Regeln" genannt ist, sind die Begriffe "Steuern", "gesteuertes Element" usw. hierin, sofern es nicht anders beschrieben oder aus dem Kontext anders zu verstehen ist, als "Steuern oder Regeln", "gesteuertes oder geregeltes Element" usw. zu verstehen. FIG. 1A, 1B und 1C und FIG. 2A bis FIG. 2E zeigen jeweils eine schematische Darstellung eines Haarbehandlungssystems 200 gemäß verschiedenen Ausführungsbeispielen, wobei unterschiedliche Ausführungsbeispiele mit nachgestellten Kleinbuchstaben gekennzeichnet sind.

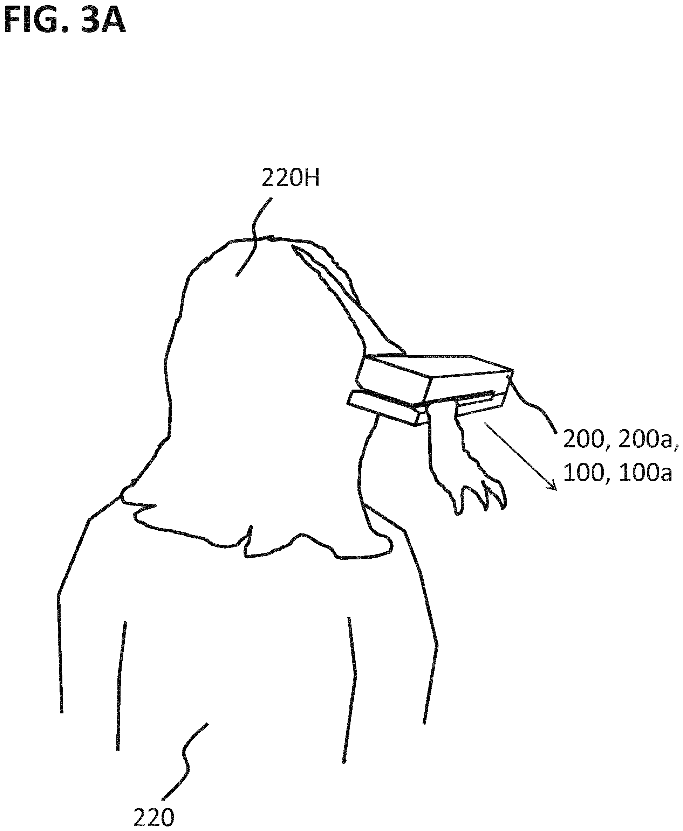

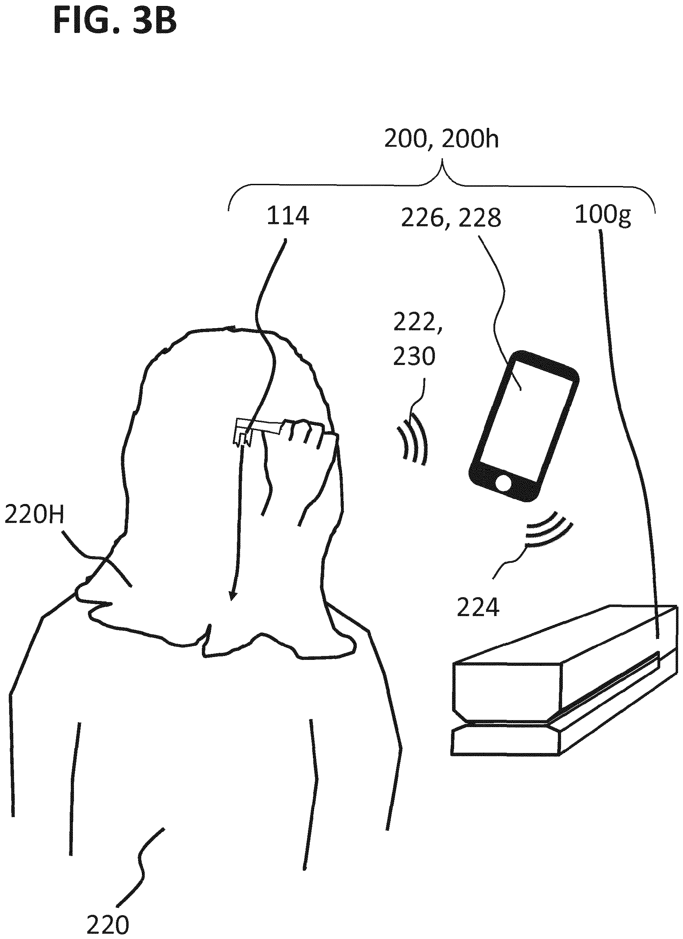

FIG. 3A und 3B zeigen jeweils eine schematische Darstellung einer Anwendung eines Haarbehandlungssystems 200 gemäß verschiedenen Ausführungsbeispielen.

In verschiedenen Ausführungsbeispielen weist das Haarbehandlungssystem 200 eine Haarbehandlungsvorrichtung 100 (verschiedene Ausführungsbeispiele sind als 100a, 100b usw. gekennzeichnet) auf.

Auch wenn in den Figuren die Haarbehandlungsvorrichtung 100 schematisch als Glätteisen (FIG. 1A, 1B, 2A bis 2F) oder als Lockenstab (FIG. 1C) dargestellt ist, ist zu verstehen, dass das jeweilige Ausführungsbeispiel auch mittels der anderen Art von Haarbehandlungsvorrichtung oder einer ähnlichen Haarbehandlungsvorrichtung (welche dafür vorgesehen ist, am Haar mechanische Arbeit zu verrichten und das Haar dabei zu erhitzen, um eine semipermanente Umformung des Haars zu bewirken) zu verwirklichen sein kann, sofern nicht aus dem Kontext etwas anderes hervorgeht.

Die Haarbehandlungsvorrichtung 100 kann einen Vorrichtungskörper 100K aufweisen. Der Vorrichtungskörper 100K kann aus einem festen Material, z.B. Kunststoff oder Metall, gebildet sein oder ein solches Material aufweisen. Beispielsweise kann der Vorrichtungskörper 100K aus einem Material gebildet sein oder ein Material aufweisen, welches üblicherweise für ein Glätteisen oder einen Lockenstab verwendet wird.

In verschiedenen Ausführungsbeispielen kann die Haarbehandlungsvorrichtung 100 ferner eine steuer- oder regelbare Heizvorrichtung 122 aufweisen (nur in FIG 1C dargestellt, sonst im Inneren der Haarbehandlungsvorrichtung 100 verborgen), welche beispielsweise in den Vorrichtungskörper 100K integriert sein kann.

In verschiedenen Ausführungsbeispielen kann das Haarbehandlungssystem 200 eine Sensorvorrichtung 114 mit mindestens einem Sensor 106 aufweisen zum Erfassen mindestens eines Haarzustandsparameters und/oder mindestens eines Umgebungsparameters. Der mindestens eine Sensor 106 kann einen oder mehrere der oben beschriebenen Sensoren 106 und/oder andere/weitere Sensoren 106 aufweisen.

Der mindestens eine Haarzustandsparameter kann, wie oben ausgeführt, z.B. eine Haarfeuchtigkeit, einen Haarschädigungsgrad, eine Haardicke, eine Haardichte, eine Lockigkeit oder ähnliches aufweisen. Der Umgebungsparameter kann, wie oben ausgeführt, z.B. eine Luftfeuchtigkeit, eine Temperatur, eine Windstärke oder ähnliches aufweisen.

In verschiedenen Ausführungsbeispielen kann der mindestens eine Sensor 106 eingerichtet sein, mehr als einen Parameter zu erfassen, beispielsweise kann der Sensor 106 ein NIR-Spektrometer aufweisen, welches eingerichtet sein kann, sowohl Parameter zum Ermitteln der Haarfeuchtigkeit als auch Parameter zum Ermitteln des Haarschädigungsgrads zu erfassen, und/oder der Sensor 106 kann eine Kamera aufweisen, welche (z.B. mittels einer Software) eingerichtet sein kann, Parameter zum Ermitteln der Lockigkeit zu ermitteln.

In verschiedenen Ausführungsbeispielen, wie in FIG. 1A bis 1C und 2A bis 2C dargestellt, kann der Sensor 106 im Vorrichtungskörper 100K angeordnet sein, beispielsweise versiegelt eingebaut sein. Damit kann ermöglicht sein, dass die Haarbehandlungsvorrichtung 100 unempfindlich ist gegen Feuchtigkeit (insbesondere auch gegen eine Feuchtigkeit des aufzubringenden Haarbehandlungsmittels) und Schmutz.

Ferner kann mittels des Versiegelns erreicht werden, dass die Haarbehandlungsvorrichtung gereinigt werden kann, ohne den Sensor 106 oder eine andere Vorrichtung zu beschädigen. Der Sensor 106 kann beispielsweise bei einem Spritzgießen des Vorrichtungskörpers 100K mit eingegossen werden.

In verschiedenen Ausführungsbeispielen, z.B. wenn der mindestens eine Sensor 106 einen optischen Sensor aufweist oder daraus besteht, kann der Vorrichtungskörper 100K zwischen dem Sensor 106 und einer Oberfläche des Vorrichtungskörpers 100K transparent sein. In verschiedenen Ausführungsbeispielen kann, sofern das zweckdienlich ist, der Sensor 106 derart im Vorrichtungskörper 100 angeordnet sein, dass er dem Haar 220H des Nutzers 220 bei einer üblichen Anordnung der Haare an oder in der Haarbehandlungsvorrichtung 100 zugewandt ist oder mit dem Haar 220H in Kontakt ist.

In verschiedenen Ausführungsbeispielen, wie in FIG. 1A bis 1C und 2A bis 2C dargestellt, kann der Sensor 106 im Vorrichtungskörper 100K angeordnet sein.

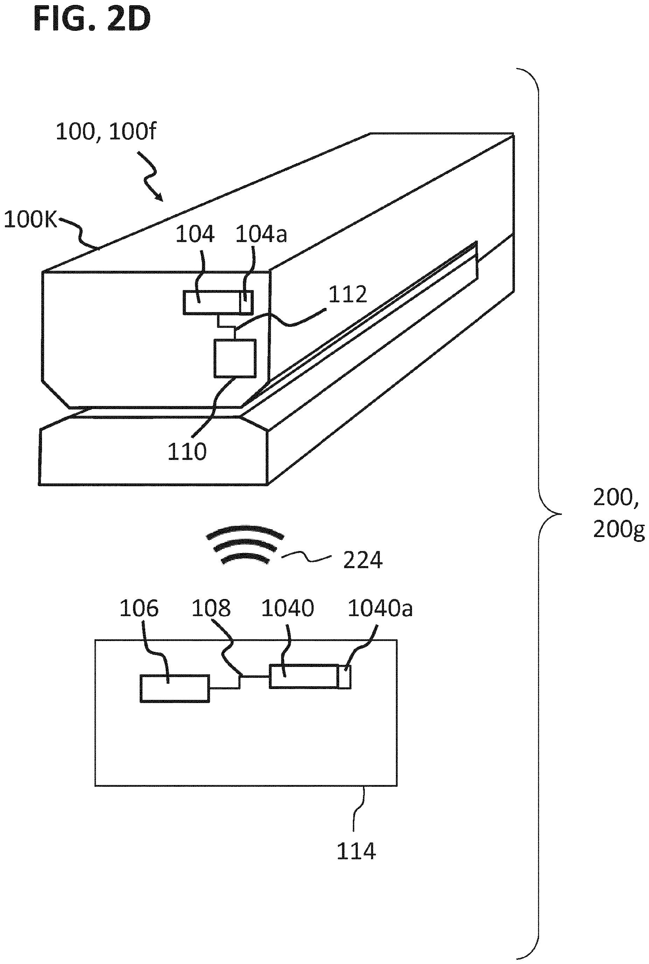

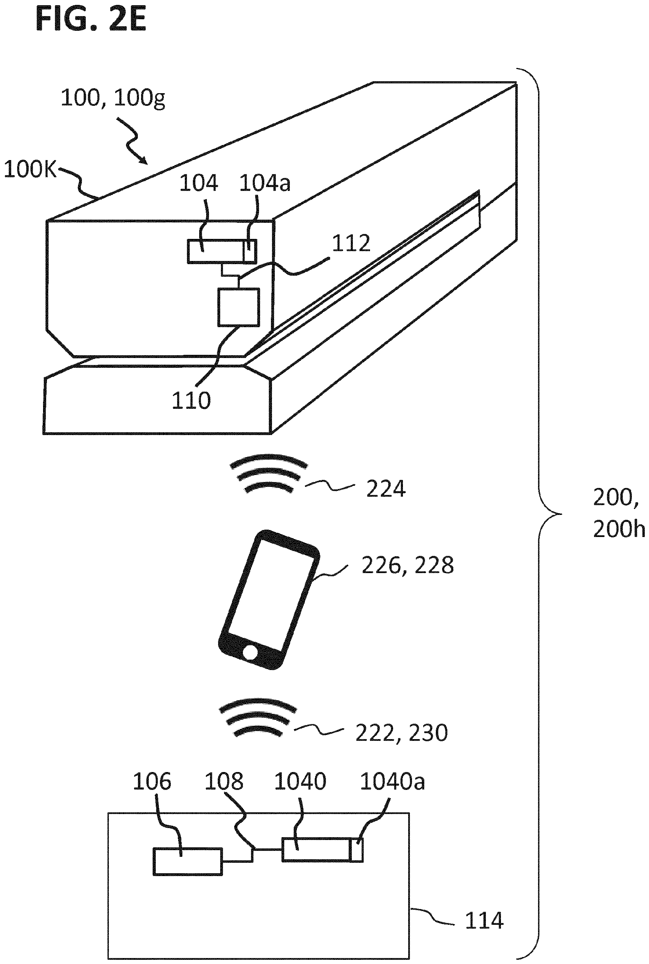

In verschiedenen Ausführungsbeispielen, wie in FIG. 2D bis 2F dargestellt, kann der Sensor 106 nicht in der Haarbehandlungsvorrichtung 100 integriert sein, sondern Teil einer separaten Sensorvorrichtung 114 sein. Die separate Sensorvorrichtung 114 kann, wie in FIG. 2D und FIG. 2E dargestellt, in verschiedenen Ausführungsbeispielen allein dem Erfassen, ggf. Bearbeiten und Übermitteln der erfassten und/oder bearbeiteten Daten dienen.

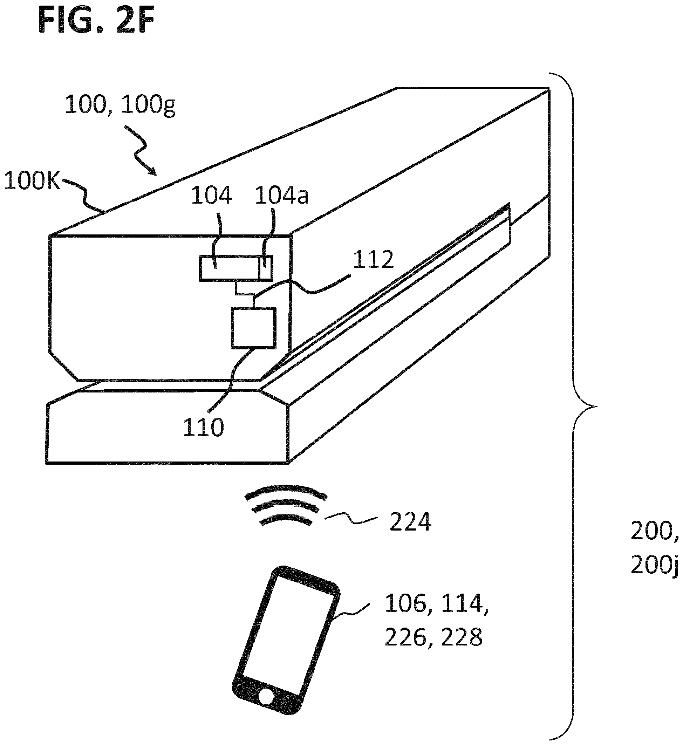

In verschiedenen Ausführungsbeispielen kann die Sensorvorrichtung 114 außerdem mindestens eine weitere Funktion erfüllen, d.h. die Sensorvorrichtung 114 kann, wie in FIG. 2F beispielhaft dargestellt, mit einer weiteren Vorrichtung des Systems integriert sein, beispielsweise mit einer Ein-und/oder Ausgabevorrichtung. In FIG. 2F kann ein Smartphone beispielsweise als Sensor genutzt werden, z.B. mittels seiner Kamera, und sein berührungsfähiger Bildschirm kann als Ein- und Ausgabevorrichtung genutzt werden. Außerdem kann die elektronische Schaltkreisvorrichtung des Smartphones, die eine Prozessor und einen Speicher aufweisen kann, eingerichtet sein, eine Auswertung der aufgenommenen Daten (z.B. Bilder) vorzunehmen, und z.B. aus einem Bild, welches Haar 220H des Nutzers 220 zeigt, eine Lockigkeit des Haars o.ä. ermitteln. Der ermittelte Haarzustandsparameter kann der Haarbehandlungsvorrichtung übermittelt werden zum Steuern oder Regeln der Haarbehandlungsvorrichtung.

Die externe Sensorvorrichtung 114 kann eine elektronische Schaltkreisvorrichtung 1040 und eine kabellose Datenaustauschvorrichtung 1040a aufweisen, welche ähnlich oder gleich der elektronischen Schaltkreisvorrichtung 104 und der kabellose Datenaustauschvorrichtung 104a aus den vorherigen Ausführungsbeispielen sein können.

In verschiedenen Ausführungsbeispielen kann die Haarbehandlungsvorrichtung 100 eine im oder am Vorrichtungskörper 100K angeordnete elektronische Schaltkreisvorrichtung 104 aufweisen.

Die elektronische Schaltkreisvorrichtung 104 kann in verschiedenen Ausführungsbeispielen mit dem mindestens einen Sensor 106 gekoppelt sein, beispielsweise mittels einer Verbindung 108, zum Empfangen des erfassten Sensorwerts. Beim Vorhandensein einer Mehrzahl von Sensoren 106 kann die elektronische Schaltkreisvorrichtung 104 eine eigene Kopplung zu jedem der Sensoren 106 aufweisen. Die Kopplung kann in verschiedenen Ausführungsbeispielen eine elektrisch leitende Verbindung, eine (Glas-)faserverbindung und/oder eine kabellose Verbindung aufweisen oder sein. Die elektronische Schaltkreisvorrichtung 104 kann zum Empfangen des mindestens einen Sensorwerts von dem mindestens einen Sensor 106 eingerichtet sein.

In verschiedenen Ausführungsbeispielen kann die elektronische Schaltkreisvorrichtung 104 eine Datenverarbeitungsvorrichtung sein oder aufweisen, z.B. kann sie mit einem Speicher und einem Prozessor, z.B. einem Mikroprozessor, ausgerüstet sein, welche eingerichtet sein können, z.B. mittels einer Programmierung, die Daten vom Sensor 106 zu empfangen und daraus einen oder mehrere Haarbehandlungsparameter abzuleiten zum Steuern oder Regeln der Haarbehandlungsvorrichtung, z.B. wie oben beschrieben mittels Steuerns oder Regelns einer Temperatur des Glätteisens/Lockenstabs, einer auf das Haar 220H des Nutzers 220 ausgeübten Kraft (z.B. in Form eines Drucks, mit welchem zwei Teile einer zweiteiligen Haarbehandlungsvorrichtung, wie sie in allen Ausführungsbeispielen von FIG. 1A bis 1C, 2A bis 2F, 3A und 3B erkennbar sind, aufeinandergepresst werden), und/oder einer Dosierung eines Haarbehandlungsmittels.

Die thermischen/mechanischen Elemente der Haarbehandlungsvorrichtung können in verschiedenen Ausführungsbeispielen jeweils mittels eines Aktuators 110 gesteuert oder geregelt sein oder werden.

Beispielsweise kann die Heizvorrichtung 122 als Aktuator 110 eine Temperatursteuerung/-regelung aufweisen, das Ausüben der Kraft auf das Haar kann mittels eines steuer-/regelbaren Motors als Aktuator 110 erfolgen, und/oder die Aufbringvorrichtung (Düsen 120 der Aufbringvorrichtung sind in FIG. 1B dargestellt) für das Haarbehandlungsmittel kann als Aktuator 110 eine Pumpe und/oder ein Dosierventil aufweisen. Der Aktuator 110 kann somit eingerichtet sein, einen Haarbehandlungsparameter zu beeinflussen. Der Aktuator 110 kann jeweils so gesteuert oder geregelt sein oder werden, dass der mittels des Aktuators 110 beeinflusste Haarbehandlungsparameter dem anhand des Haarzustandsparameters und/oder des Umgebungsparameters ermittelten Haarbehandlungsparameter entspricht.

Die elektronische Schaltkreisvorrichtung 104 kann in verschiedenen Ausführungsbeispielen eingerichtet sein, basierend auf den empfangenen Daten vom Sensor 106 mindestens eine Empfehlung zu ermitteln und dem Nutzer 220 bereitzustellen, beispielsweise, wie oben beschrieben, als Anleitungsvideo, als Produktempfehlung für ein bekanntes Haarbehandlungsmittel oder als Rezept für ein individuelles Haarbehandlungsmittel wie oben beschrieben.

In verschiedenen Ausführungsbeispielen, z.B. in einem Fall, in welchem der mindestens eine Sensor 106 genutzt wird, um einen Haarschädigungsgrad, eine Lockigkeit oder ähnliches zu ermitteln, kann die Haarbehandlungsvorrichtung 100 eingerichtet sein, den Aktuator 110 zu steuern, z.B. eine dem Haarschädigungsgrad, der Lockigkeit o.ä. entsprechende Temperatur für die Haarbehandlung, z.B. einen Glättvorgang oder einen Lockungsvorgang, einzustellen.

In verschiedenen Ausführungsbeispielen, z.B. in einem Fall, in welchem der mindestens eine Sensor ferner mindestens einen Behandlungssensor 128 aufweist, z.B. Temperatursensoren 128, um eine Haartemperatur zu ermitteln, z.B. an Positionen vor und hinter der Heizvorrichtung, kann die Haarbehandlungsvorrichtung 100 eingerichtet sein, den Aktuator 110 zu regeln, z.B. eine dem Haarschädigungsgrad, der Lockigkeit o.ä. entsprechende Temperatur für die Haarbehandlung, z.B. einen Glättvorgang oder einen Lockungsvorgang, einzustellen und anhand der mittels der Behandlungssensoren 128 erfassten Temperatur (z.B. insbesondere der Temperatur hinter der Heizvorrichtung, d.h. nach der Hitzebehandlung) nachzuregeln. Andere mögliche Arten von Behandlungssensoren 128 sind oben beschrieben.

In verschiedenen Ausführungsbeispielen kann die Haarbehandlungsvorrichtung 100 eingerichtet sein, den Aktuator 110 allein aufgrund von Temperatursensordaten zu regeln.

In verschiedenen Ausführungsbeispielen kann der Aktuator 110 eingerichtet sein, auf eine relativ hohe Temperatur, z.B. in einem Bereich von etwa 200°C bis etwa 230°C, eingestellt zu werden, wenn ermittelt wird, dass das Haar 220H des Nutzers 220 beispielsweise ungeschädigt und/oder dick ist, und auf eine relativ niedrige Temperatur eingestellt zu werden, z.B. in einem Bereich von etwa 150°C bis etwa 180°C, wenn ermittelt wird, dass das Haar 220H des Nutzers 220 beispielsweise vorgeschädigt und/oder dünn ist.

In verschiedenen Ausführungsbeispielen kann dem Nutzer 220 außerdem eine Haarbehandlungsempfehlung bereitgestellt werden, z.B. im Fall des Glätteisens oder Lockenstabs eine Empfehlung "jede Haarsträhne für maximal fünf Sekunden behandeln" oder ähnliches.

In verschiedenen Ausführungsbeispielen, wie in FIG. 1B dargestellt, kann der Aktuator 110, beispielsweise alternativ oder zusätzlich zur Temperatursteuerung oder -regelung, die steuer- oder regelbare Aufbringvorrichtung (auch als Dosiervorrichtung bezeichnet) aufweisen, welche eingerichtet sein kann, das Haarbehandlungsmittel basierend auf den erfassten Sensordaten zu dosieren.

Wie oben ausgeführt, kann die Aufbringvorrichtung mindestens eine Pumpe und/oder mindestens ein Ventil aufweisen, welches derart steuer- oder regelbar sein kann, dass ein Volumen oder eine Menge des Haarbehandlungsmittels dosiert werden kann. Das Dosieren kann in verschiedenen Ausführungsbeispielen in Abhängigkeit von einer Position der Haarbehandlungsvorrichtung 100 am Haar 220H erfolgen, z.B. kann bei einem Ermitteln, dass die Haarbehandlungsvorrichtung 100 sich am Haaransatz befindet, eine andere Menge des Haarbehandlungsmittels abgegeben werden als bei einem Ermitteln, dass die Haarbehandlungsvorrichtung 100 sich an den Haarspitzen befindet. Beispielsweise können die Haarspitzen eine größere Menge eines Haarpflegemittels erfordern als der Haaransatz.

Wie in FIG. 1B dargestellt ist, kann in verschiedenen Ausführungsbeispielen das Haarbehandlungsmittel in der Haarbehandlungsvorrichtung 100 in einer Kartusche 130 aufgenommen sein oder werden. Die Kartusche 130 kann beispielsweise wiederbefüllbar sein. Die Kartusche 130 kann in verschiedenen Ausführungsbeispielen in die Haarbehandlungsvorrichtung 100 einbringbar sein, z.B. mittels einer Öffnung 116.

Das Dosierventil kann in verschiedenen Ausführungsbeispielen Teil der Haarbehandlungsvorrichtung 100 sein. Alternativ kann das Dosierventil Teil der Kartusche 130 sein.

In verschiedenen Ausführungsbeispielen kann, statt die Kartusche 130 zu verwenden, das Haarbehandlungsmittel direkt in einen Aufnahmeraum der Haarbehandlungsvorrichtung 100 einbringbar sein.

In verschiedenen Ausführungsbeispielen kann das Haarbehandlungssystem 200 über eine Mischvorrichtung 132 verfügen, mittels welcher das personalisierte Haarbehandlungsmittel (wie oben beschrieben) zubereitbar sein kann. In verschiedenen Ausführungsbeispielen kann das personalisierte Haarbehandlungsmittel mittels einer nicht zum Haarbehandlungssystem 200 gehörenden Mischvorrichtung angefertigt und in der Kartusche bereitgestellt werden, beispielsweise in einem Kosmetiksalon, bei einem sonstigen Hersteller, z.B. bei einem Internethändler, o.ä.

Bei dem Haarbehandlungssystem 200, 200a aus FIG. 1A kann in verschiedenen Ausführungsbeispielen die Sensorvorrichtung, in diesem Fall der mindestens eine Sensor 106, in die beispielhaft als Glätteisen ausgeführte Haarbehandlungsvorrichtung 100, 100a integriert sein. Die Haarbehandlungsvorrichtung 100a kann in verschiedenen Ausführungsbeispielen eingerichtet sein, mittels der integrierten elektronischen Schaltkreisvorrichtung 104 basierend auf dem mittels des integrierten Sensors 106 erfassten Haarzustandsparameter und/oder Umgebungsparameter mindestens einen Haar-Behandlungsparameter zu ermitteln und die Haarbehandlung mittels des mindestens einen Aktuators 110 zu steuern, wobei die Haarbehandlung ein Aufbringen des Haar-Behandlungsmittels und eine mechanische und/oder thermische Behandlung (d.h. Pressen und/oder Erhitzen des Haars 220H) aufweist.

Das Haarbehandlungssystem 200, 200b aus FIG. 1B kann in verschiedenen Ausführungsbeispielen dem Haarbehandlungssystem 200, 200a aus FIG. 1A entsprechen, und außerdem noch über die Mischvorrichtung 132 verfügen, welche Teil der Haarbehandlungsvorrichtung 100, 100a sein kann oder eine separate Vorrichtung sein kann.

Darüber hinaus ist die Haarbehandlungsvorrichtung 100, 100a aufgeklappt dargestellt, so dass die Heizvorrichtung 122, Öffnungen der Dosiervorrichtung 120 und Behandlungssensoren 128 sichtbar sind.

Das Haarbehandlungssystem 200, 200c aus FIG. 1C kann in verschiedenen Ausführungsbeispielen dem Haarbehandlungssystem 200, 200a aus FIG. 1A entsprechen, abgesehen davon, dass die Haarbehandlungsvorrichtung 100, 100b beispielhaft als Lockenstab 100, 100b dargestellt ist.

Das Haarbehandlungssystem 200, 200d aus FIG. 2A kann sich in verschiedenen Ausführungsbeispielen vom Haarbehandlungssystem 200a aus FIG. 1A dahingehend unterscheiden, dass die Haarbehandlungsvorrichtung 100c eine Datenaustauschvorrichtung 104a aufweist, welche eingerichtet sein kann, Daten kabellos zu übertragen und zu empfangen (dargestellt als Signale 224 und 230).

Das Haarbehandlungssystem 200, 200d kann in verschiedenen Ausführungsbeispielen eingerichtet sein, Daten mittels der Datenaustauschvorrichtung 104a mit einer externen Datenverarbeitungsvorrichtung 226 auszutauschen. Die externe Datenverarbeitungsvorrichtung kann beispielsweise ein externer Computer, z.B. eine Cloud sein.

Die externe Datenverarbeitungsvorrichtung 226 kann, wie oben beschrieben, eingerichtet sein, die erfassten Sensordaten von dem Haarbehandlungssystem 200d zu empfangen, die Steuerungs-oder Regelungsparameter und/oder die mindestens eine Empfehlung zu ermitteln und diese dem Haarbehandlungssystem 200c zurück zu übermitteln. Das heißt, das Haarbehandlungssystem 200d kann, wie oben beschrieben, eingerichtet sein, die Steuerungs- oder Regelungsparameter und/oder die mindestens eine Empfehlung indirekt zu ermitteln.

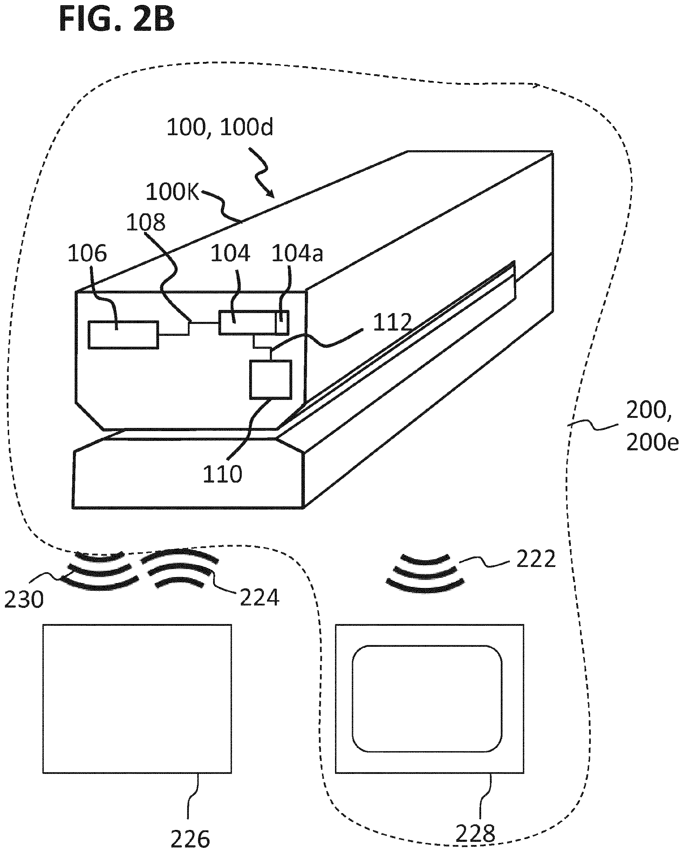

Das Haarbehandlungssystem 200, 200e aus FIG. 2B kann in verschiedenen Ausführungsbeispielen im Wesentlichen dem Haarbehandlungssystem 200d aus FIG. 2A entsprechen, aber um eine Ausgabevorrichtung 228 ergänzt sein. Beispielhaft ist ein Bildschirm, d.h. eine Anzeigevorrichtung als Ausgabevorrichtung 228 dargestellt. Alternativ oder zusätzlich kann das Haarbehandlungssystem 200e allerdings über eine beliebige Art der Ausgabevorrichtung 228 verfügen, beispielsweise wie oben beschrieben.

Die Ausgabevorrichtung 228 kann in verschiedenen Ausführungsbeispielen eingerichtet sein, Daten (als (z.B. kabelloses) Signal 222) von der Haarbehandlungsvorrichtung 100d zu empfangen und auszugeben, z.B. anzuzeigen, beispielsweise eine mittels der elektronischen Schaltkreisvorrichtung 104 und/oder der externen Datenverarbeitungsvorrichtung 226 ermittelte Empfehlung, z.B. eine Haarbehandlungsempfehlung (z.B. wie oben beschrieben eine Schritt-für-Schritt-Anleitung o.ä.) und/oder ein empfohlenes Haarpflegemittel oder Ähnliches.

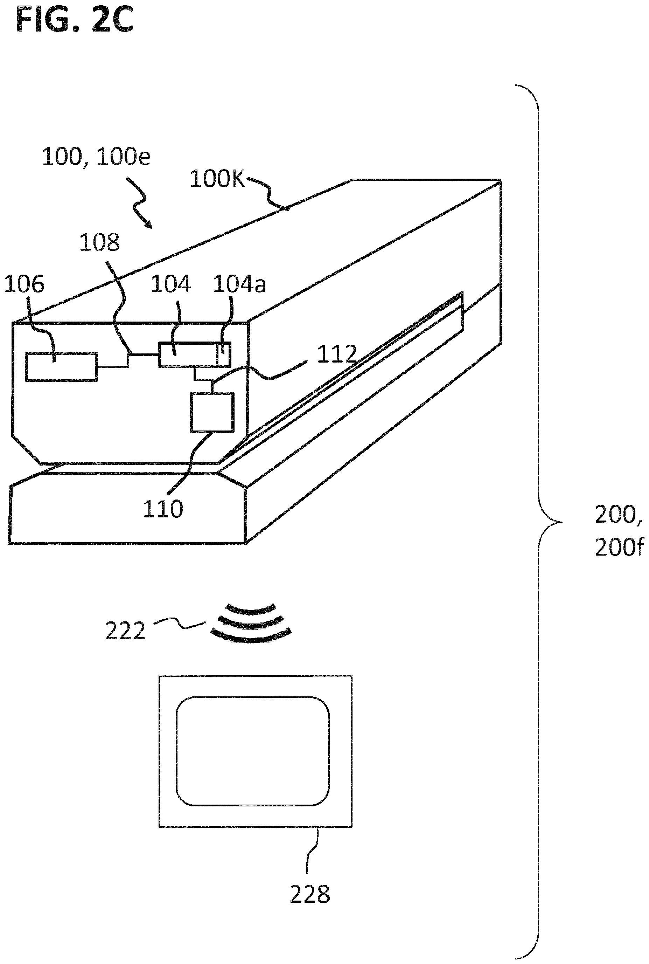

Das Haarbehandlungssystem 200, 200f aus FIG. 2C kann in verschiedenen Ausführungsbeispielen eine Haarbehandlungsvorrichtung 100, 100e aufweisen, welche in wesentlichen Teilen ähnlich oder identisch der Haarbehandlungsvorrichtung 100a sein kann.

Die Haarbehandlungsvorrichtung 100e kann gegenüber der Haarbehandlungsvorrichtung 100a allerdings um eine Datenaustauschvorrichtung 104a ergänzt sein. Die Datenaustauschvorrichtung 104a kann, ähnlich der Datenaustauschvorrichtung 104a aus FIG. 2A und/oder Fig. 2B, in verschiedenen Ausführungsbeispielen eingerichtet sein, Daten 224, 230 kabellos zu übertragen und zu empfangen.

Das Haarbehandlungssystem 200, 200f kann in verschiedenen Ausführungsbeispielen eine Anzeigevorrichtung 228 aufweisen.

Die Anzeigevorrichtung 228 kann in verschiedenen Ausführungsbeispielen eingerichtet sein, Daten (als Signal 222) von der Haarbehandlungsvorrichtung 100e zu empfangen und auszugeben, z.B. wie oben bei FIG. 2B beschrieben.

Das Haarbehandlungssystem 200, 200g aus FIG. 2D kann in verschiedenen Ausführungsbeispielen eine Haarbehandlungsvorrichtung 100, 100f aufweisen, welche in wesentlichen Teilen ähnlich oder identisch der Haarbehandlungsvorrichtung 100a sein kann.

Im Unterschied zur Haarbehandlungsvorrichtung 100a kann der mindestens eine Sensor 106 allerdings, statt in die Haarbehandlungsvorrichtung 100f integriert zu sein, Teil einer separaten Sensorvorrichtung 114 sein. Zum Empfangen der Sensordaten von der Sensorvorrichtung 114 kann die Haarbehandlungsvorrichtung 100f in verschiedenen Ausführungsbeispielen eine Datenaustauschvorrichtung 104a aufweisen.

Die Datenaustauschvorrichtung 104a der Haarbehandlungsvorrichtung 100, 100f kann in verschiedenen Ausführungsbeispielen eingerichtet sein, Daten 224 kabellos zu übertragen und zu empfangen.

Eine elektronische Schaltkreisvorrichtung 1040 kann in verschiedenen Ausführungsbeispielen mit dem mindestens einen Sensor 106 gekoppelt sein mittels einer Datenverbindung 108, welche ähnlich oder gleich der Datenverbindung 108 aus den vorherigen Ausführungsbeispielen sein kann.

Die externe Sensorvorrichtung 114 kann eingerichtet sein, der Haarbehandlungsvorrichtung 100f die erfassten Sensordaten und/oder anhand der Sensordaten (z.B. mittels der Schaltkreisvorrichtung 1040) ermittelte Steuer- oder Regelungsparameter und/oder Empfehlungen zu übermitteln (dargestellt als Signal 224), beispielsweise mittels einer Datenaustauschvorrichtung 1040a. Die externe Sensorvorrichtung 114 kann beispielsweise ein Smartphone oder Ähnliches aufweisen, wobei beispielsweise eine integrierte Kamera als Sensor genutzt werden kann, oder eine andere Art von Sensorvorrichtung, z.B. ein (NIR-)Spektrometer, akustischer Kamm, etc., beispielsweise wie oben beschrieben.

Die Haarbehandlungsvorrichtung 100f kann eingerichtet sein, den Aktuator 110 anhand der empfangenen Steuer- oder Regelungsparameter zu steuern oder zu regeln und/oder anhand der empfangenen Sensordaten Steuer- oder Regelungsparameter zu ermitteln (z.B. mittels der elektronischen Schaltkreisvorrichtung 104) und anhand der ermittelten Steuer- oder Regelungsparameter den Aktuator 110 zu steuern oder zu regeln.

Das Haarbehandlungssystem 200, 200g aus FIG. 2E kann in verschiedenen Ausführungsbeispielen eine Haarbehandlungsvorrichtung 100, 100h aufweisen, welche in wesentlichen Teilen ähnlich oder identisch der Haarbehandlungsvorrichtung 100d aus FIG. 2B sein kann.

Im Unterschied zum Haarbehandlungssystem 200e aus FIG. 2B können die Haarbehandlungsvorrichtung 100h und die externe Sensorvorrichtung 114 eingerichtet sein, ihre Daten nicht direkt miteinander auszutauschen, sondern mittels einer kombinierten Datenverarbeitungs- und Anzeigevorrichtung 226, 228 (z.B. eines Smartphones, eines Tablet, eines Laptops o.ä.). Anders ausgedrückt kann die kombinierte Datenverarbeitungs- und Anzeigevorrichtung 226, 228 als Herzstück des Haarbehandlungssystems 200g fungieren. Auf der kombinierten Datenverarbeitungs- und Anzeigevorrichtung 226, 228 kann beispielsweise eine App oder ein Programm installiert sein, welches eingerichtet sein kann, das Erfassen der Sensordaten und das Steuern/Regeln der Haarbehandlungsparameter zu verwalten, ggf. selbst, z.B. alternativ oder zusätzlich zur elektronischen Schaltkreisvorrichtung 104 und/oder der elektronischen Schaltkreisvorrichtung 1040, die Steuerungs- oder Regelungsparameter und/oder die Empfehlung zu ermitteln und/oder, ggf. alternativ oder zusätzlich zur Haarbehandlungsvorrichtung 100h, die Empfehlung bereitzustellen, z.B. mittels der Ausgabevorrichtung 228, z.B. als Anzeige oder beispielsweise akustisch, z.B. mittels eines Lautsprechers.

In verschiedenen Ausführungsbeispielen kann die kombinierte Datenverarbeitungs- und Anzeigevorrichtung 226, 228 ferner eingerichtet sein, die Sensordaten einer (weiteren) externen Datenverarbeitungsvorrichtung 226 bereitzustellen, z.B. einer Cloud, und von der (weiteren) externen Datenverarbeitungsvorrichtung 226 die von dieser empfangenen Steuerungs- oder Regelungsparameter und/oder die Empfehlung der Haarbehandlungsvorrichtung 100f zu übermitteln und/oder die Empfehlung bereitzustellen.

In verschiedenen Ausführungsbeispielen kann zum Ermitteln eines Haarzustands, z.B. eines Haarschädigungsgrads o.ä. und/oder eines Umgebungsparameters, wie oben beschrieben eine Datenbank genutzt werden, in welcher den Sensordaten oder ggf. Kombinationen von Sensordaten, die Haarzustände (z.B. Haarschädigungsgrade) oder Umgebungsparameter zugeordnet sein können. Die Datenbank kann vorab mittels Versuchen erstellt worden sein und z.B. in der Datenverarbeitungs- und Anzeigevorrichtung 226, 228 und/oder in mindestens einer der elektronischen Schaltkreisvorrichtungen 104, 1040 gespeichert sein, und/oder kann fortwährend, z.B. bei Nutzung einer Cloud, mittels Nutzerdaten erstellt oder ergänzt werden und dem Haarbehandlungssystem 200g bereitgestellt werden.

In verschiedenen Ausführungsbeispielen kann die Datenbank ferner eine Behandlungs- und/oder Produktempfehlung auf Basis des Haarzustands, z.B. des Haarschädigungsgrads, und/oder auf Basis des mindestens einen Umgebungsparameters bereitstellen. Bei dem Ermitteln der Behandlungs- und/oder Produktempfehlung kann ferner, beispielsweise wie oben beschrieben, weitere vom Nutzer 220 bereitgestellte Information einbezogen werden, z.B. ein Wunschzustand oder Ähnliches.

Die erfassten (gemessenen) Sensorwerte können, wie oben beschrieben, entweder direkt in der Haarbehandlungsvorrichtung 100 ausgewertet werden, z.B. mittels der elektronischen Schaltkreisvorrichtung 104, oder indirekt ausgewertet werden, indem sie an eine externe Datenverarbeitungsvorrichtung 226 übertragen werden, um dort ausgewertet zu werden, z.B. wie oben beschrieben. Dabei können die Sensordaten oder Teile der Sensordaten in verschiedenen Ausführungsbeispielen ausgewertet werden mittels eines Vergleichs mit (z.B. empirisch gewonnenen) Datenbankeinträgen.

In verschiedenen Ausführungsbeispielen kann das ermittelte Haarbehandlungsmittel, z.B. Haarstylingmittel, mittels der Haarbehandlungsvorrichtung oder des Haarbehandlungssystems auf das Haar aufgebracht werden, wobei Steuerungs- oder Regelungsparameter zum Dosieren des Haarbehandlungsmittels mittels der elektronischen Schaltkreisvorrichtung 104, der elektronischen Schaltkreisvorrichtung 1040 und/oder der externen Datenverarbeitungsvorrichtung 226 und/oder der weiteren externen Datenverarbeitungsvorrichtung bereitgestellt sein oder werden können.

Das Haarbehandlungssystem 200, 200j aus FIG. 2F kann in verschiedenen Ausführungsbeispielen eine Haarbehandlungsvorrichtung 100, 100g aufweisen, welche in wesentlichen Teilen ähnlich oder identisch der Haarbehandlungsvorrichtung 100f aus FIG. 2D sein kann.

Die separate Sensorvorrichtung 114 kann, wie im Zusammenhang mit FIG. 2D bereits beschrieben, ein Smartphone, ein Tablet, einen Laptop oder Ähnliches aufweisen, beispielsweise kann die Kamera und/oder ein Mikrofon des Smartphones/Tablets/Laptops 114 als Sensor 106 oder Teil des Sensors genutzt werden. Dank eines berührungsempfindlichen Bildschirms oder einer Kombination von Tastatur und Bildschirm und/oder Maus und Bildschirm kann die Sensorvorrichtung 114 des Haarbehandlungssystems 200j allerdings gleichzeitig als Eingabe-und/oder Ausgabevorrichtung 228 dienen, und ein Prozessor des Smartphones/Tablets/Laptops kann als die elektronische Schaltkreisvorrichtung 1040 der Sensorvorrichtung 114 oder als die Datenverarbeitungsvorrichtung 226 dienen, z.B. mittels einer dort installierten Software (z.B. App), und Funktionen bereitstellen wie oben für die elektronische Schaltkreisvorrichtung 1040 der Sensorvorrichtung 114 oder als die Datenverarbeitungsvorrichtung 226 beschrieben.

FIG. 3A zeigt eine schematische Darstellung einer Anwendung eines Haarbehandlungssystems 200, 200a gemäß verschiedenen Ausführungsbeispielen, und FIG. 3B zeigt eine schematische Darstellung einer Anwendung eines Haarbehandlungssystems 200, 200h gemäß verschiedenen Ausführungsbeispielen.

Wie in FIG. 3A dargestellt ist, kann die Haarbehandlungsvorrichtung 100a auf für die Haarbehandlungsvorrichtung übliche Weise zur Haarbehandlung genutzt werden. Dargestellt ist ein Glätteisen, in welches die Haare 220H strähnenweise eingeklemmt werden und welches typischerweise in Pfeilrichtung, d.h. vom Haaransatz in Richtung zu den Haarspitzen, bewegt wird. Sinngemäß wird beispielsweise bei Verwendung eines Lockenstabs als Haarbehandlungsvorrichtung das Haar strähnenweise auf die Haarbehandlungsvorrichtung 100 aufgewickelt.

Im Unterschied zum herkömmlichen Haarbehandlungssystem kann Haarbehandlungssystem 200a gemäß verschiedenen Ausführungsbeispielen eingerichtet sein, während der Haarbehandlung, z.B. wie oben beschrieben, mittels mindestens eines Sensors 106 mindestens einen Sensorwert zu erfassen und mindestens einen Haarbehandlungsparameter zu steuern oder zu regeln, wobei die Haarbehandlung zumindest das Aufbringen eines Haarbehandlungsmittels während der Haarbehandlung und mindestens eines von einem Ausüben einer Kraft auf das Haar 220H und einem Erhitzen des Haars 220H aufweist.

Wie in FIG. 3B dargestellt ist, kann das Haarbehandlungssystem 200h für eine Haarbehandlung genutzt werden. Eine externe Sensorvorrichtung 114 kann, wie oben beschrieben, genutzt werden, um mindestens einen Haarzustandsparameter, z.B. einen Haarschädigungsgrad o.ä., und/oder einen Umgebungsparameter zu ermitteln. Dafür kann die externe Sensorvorrichtung 114 in optischen und/oder körperlichen Kontakt mit dem Haar 220H des Nutzers 220 gebracht werden. Dargestellt ist eine Verwendung eines Mikrofonkamms 114 als Sensorvorrichtung zum Ermitteln des Haarschädigungsgrads.

Wie oben beschrieben kann der mindestens eine Sensorwert und/oder ein daraus ermittelter Haarzustandsparameter und/oder ein Steuerungs- oder Regelungsparameter der kombinierten Datenverarbeitungs-/Anzeigevorrichtung 226, 228 übermittelt werden (dargestellt als Signal 222, 230), und der mindestens eine Sensorwert und/oder ein daraus ermittelter Haarzustandsparameter und/oder ein Steuerungs- oder Regelungsparameter kann der Haarbehandlungsvorrichtung 100g übermittelt werden, z.B. zum Steuern und/oder Regeln des mindestens einen Aktuators 110 und/oder zum Anzeigen der mindestens einen Empfehlung.

Dargestellt ist hier ein Übermitteln des mindestens einen Sensorwerts und/oder eines daraus ermittelten Haarzustandsparameters und/oder eines Steuerungs- oder Regelungsparameters an die Haarbehandlungsvorrichtung 100g vor einem Verwenden der Haarbehandlungsvorrichtung 100g. In verschiedenen Ausführungsbeispielen kann das Übermitteln auch gleichzeitig mit dem Verwenden erfolgen.

In verschiedenen Ausführungsbeispielen können die anderen oben beschriebenen Haarbehandlungsvorrichtungen und Haarbehandlungssysteme sinngemäß ähnlich verwendet werden wie dies hier beispielhaft für zwei Haarbehandlungssysteme beschrieben ist.

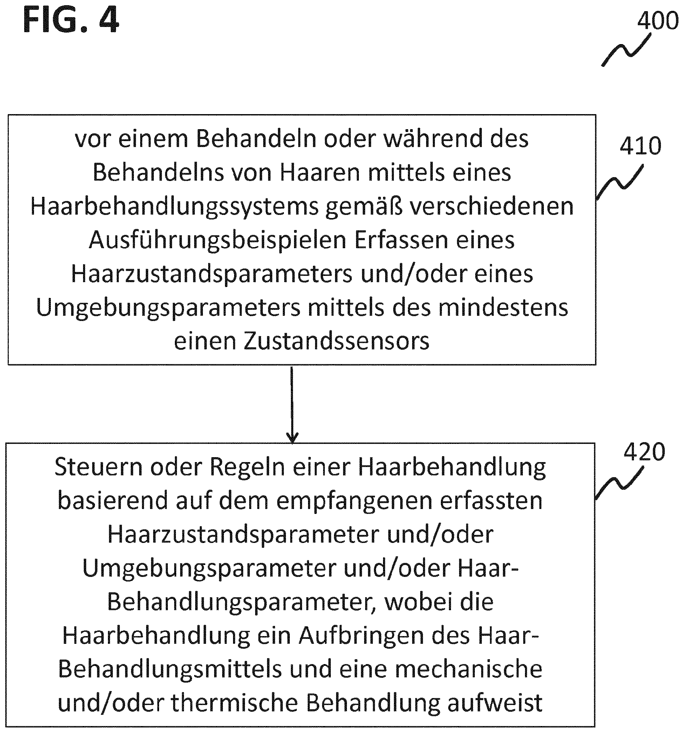

FIG. 4 zeigt ein Flussdiagramm 400 eines Verfahrens zum kosmetischen Behandeln von Haaren eines Nutzers gemäß verschiedenen Ausführungsbeispielen.

In verschiedenen Ausführungsbeispielen kann das Verfahren zum kosmetischen Behandeln von Haaren eines Nutzers aufweisen ein Erfassen eines Haarzustandsparameters und/oder eines Umgebungsparameters mittels mindestens eines Zustandssensors während eines Behandelns und/oder vor einem Behandeln von Haaren mittels eines Haarbehandlungssystems gemäß verschiedenen Ausführungsbeispielen (in 410) und ein Steuern oder Regeln einer Haarbehandlung basierend auf dem empfangenen erfassten Haarzustandsparameter und/oder Umgebungsparameter und/oder Haar-Behandlungsparameter, wobei die Haarbehandlung ein Aufbringen des Haar-Behandlungsmittels und eine mechanische und/oder thermische Behandlung aufweist (in 420).

Für die oben beschriebenen Ermittlungen kann in verschiedenen Ausführungsbeispielen eine Programmierung, z.B. eine Software genutzt werden. Dabei kann jede Software genutzt werden, die eine oben beschriebene Funktionalität bereitstellt. In verschiedenen Ausführungsbeispielen, z.B. in einem Fall, dass zum Ausführen des Verfahrens zum kosmetischen Behandeln von Haaren eines Nutzers gemäß verschiedenen Ausführungsbeispielen ein Smartphone, ein Tablet oder ähnliches genutzt wird, kann die Software als eine App bereitgestellt sein.

In verschiedenen Ausführungsbeispielen kann die in der Haarbehandlungsvorrichtung integrierte Schaltkreisvorrichtung und/oder eine externe Datenverarbeitungsvorrichtung, z.B. ein Smartphone, ein Tablet, ein Laptop, ein Smart Mirror, ein iPad, oder ähnliches geeignet sein, um bei einem Ausführen des Verfahrens zum Bereitstellen einer Haarzustandsinformation und/oder einer Umgebungszustandsinformation, z.B. bei Ermittlungsvorgängen, z.B. mittels Vergleichens mit einer Datenbank/Referenzwerten o.ä., verwendet zu werden. In verschiedenen Ausführungsbeispielen braucht die Programmierung/Software nicht auf dem Smartphone, dem Tablet, dem Laptop usw. bereitgestellt zu werden. Es kann beispielsweise ausreichend sein, wenn die in die Haarbehandlungsvorrichtung integrierte Schaltkreisvorrichtung und/oder das Smartphone o.ä. durch das Internet, mittels WLAN oder auf andere gängige Weise mit einer (z.B. einer weiteren) externen Datenverarbeitungsvorrichtung, z.B. einem Computer, beispielsweise einer Cloud, verbunden ist. In einem solchen Fall können die Berechnungen beispielsweise mittels der (weiteren) externen Datenverarbeitungsvorrichtung, z.B. mittels des Computers, ausgeführt werden, und das Ergebnis kann dem Smartphone/Tablet o.ä. und/oder der internen Schaltkreisvorrichtung bereitgestellt werden, wobei die Haarbehandlungsvorrichtung eingerichtet sein kann, die mittels der externen Datenverarbeitungsvorrichtung ermittelten Steuerungs-/Regelungsbefehle zum Steuern oder Regeln des mindestens einen Haarbehandlungsparameters einzusetzen, z.B. zum Steuern oder Regeln eines entsprechenden Aktuators.

Weitere vorteilhafte Ausgestaltungen des Verfahrens ergeben sich aus der Beschreibung der Vorrichtung und umgekehrt.

## Patentansprüche

1. Haarbehandlungssystem, aufweisend:
eine Sensorvorrichtung mit mindestens einem Zustandssensor zum Erfassen eines Haarschädigungsgrads; und
eine elektrische Haarbehandlungsvorrichtung, aufweisend:
eine Aufbringvorrichtung zum Aufbringen eines Haarbehandlungsmittels auf das Haar;
eine Haarbehandlungseinheit; und
eine elektronische Schaltkreisvorrichtung,
wobei die elektronische Schaltkreisvorrichtung mit der Sensorvorrichtung gekoppelt ist zum Empfangen des Haarschädigungsgrad, und
wobei die elektronische Schaltkreisvorrichtung eingerichtet ist, basierend auf dem empfangenen erfassten Haarschädigungsgrad die Haarbehandlung zu steuern,
wobei die Haarbehandlung ein Aufbringen des Haar-Behandlungsmittels mittels der Aufbringvorrichtung und eine mechanische und/oder thermische Behandlung mittels der Haarbehandlungseinheit aufweist,
wobei die Sensorvorrichtung mindestens einen Sensor aufweist, der eingerichtet ist zum Erfassen eines Umgebungsparameters.

2. Haarbehandlungssystem gemäß Anspruch 1,
wobei der Zustandssensor ferner zum Erfassen eines Umgebungsparameters eingerichtet ist, und wobei die elektronische Schaltkreisvorrichtung mit der Sensorvorrichtung gekoppelt ist zum Empfangen des Umgebungsparameters und/oder eines Haar-Behandlungsparameters, und
wobei die elektronische Schaltkreisvorrichtung eingerichtet ist, basierend auf dem empfangenen erfassten Umgebungsparameter und/oder Haar-Behandlungsparameter die Haarbehandlung zu steuern.

3. Haarbehandlungssystem gemäß Anspruch 1 oder 2,
wobei die Haarbehandlungsvorrichtung ein Glätteisen oder einen Lockenstab aufweist.

4. Haarbehandlungssystem gemäß einem der Ansprüche 1 bis 3,
wobei der mindestens eine Zustandssensor mindestens einen aus einer Gruppe von Zustandssensoren aufweist, die Gruppe aufweisend:
eine Kamera zum Aufnehmen eines Bildes mittels sichtbaren Lichts, UV-Lichts und/oder Nahinfrarotlichts zum Ermitteln des Haarschädigungsgrads;
ein Mikroskop zum Ermitteln des Haarschädigungsgrads und/oder einer Haardicke;
ein Spektrometer zum Aufnehmen eines Spektrums von sichtbarem Licht, UV-Licht und/oder Nahinfrarotlicht zum Ermitteln des Haarschädigungsgrads;
einen akustischen Sensor zum Ermitteln des Haarschädigungsgrads;
einen Temperatursensor zum Ermitteln einer Umgebungstemperatur;
einen Luftfeuchtigkeitssensor; und
einen Windsensor.

5. Haarbehandlungssystem gemäß Anspruch 4, wobei die Kamera weiter eingerichtet ist ein Bild mittels sichtbaren Lichts, UV-Lichts und/oder Nahinfrarotlichts aufzunehmen zum Ermitteln einer Lockigkeit, einer Haarfarbe, einer Haardicke und/oder einer Haardichte,
das Spektrometer weiter eingerichtet ist ein Spektrum von sichtbarem Licht, UV-Licht und/oder Nahinfrarotlicht aufzunehmen zum Ermitteln eines Haaraminosäuregehalts und/oder eines Haarfeuchtigkeitsgehalts.

6. Haarbehandlungssystem gemäß einem der Ansprüche 1 bis 5,
wobei die Sensorvorrichtung und die Haarbehandlungsvorrichtung separate Vorrichtungen sind.

7. Haarbehandlungssystem gemäß einem der Ansprüche 1 bis 5,
wobei zumindest ein Teil der Sensorvorrichtung und die Haarbehandlungsvorrichtung eine integrierte Einheit bilden.

8. Haarbehandlungssystem gemäß einem der Ansprüche 1 bis 7,
wobei das Haarbehandlungssystem eingerichtet ist, einen, vorzugsweise prognostizierten, Umgebungsparameter aus einer externen Quelle zu empfangen.

9. Haarbehandlungssystem gemäß einem der Ansprüche 1 bis 8,
wobei die Haarbehandlungsvorrichtung mindestens ein
gesteuertes oder geregeltes Element zum Steuern oder Regeln der Haarbehandlung aufweist.

10. Haarbehandlungssystem gemäß Anspruch 9,
wobei das gesteuerte oder geregelte Element eine Dosiervorrichtung für das Haarbehandlungsmittel und/oder, als die Haarbehandlungseinheit, eine Heizvorrichtung und/oder einen Motor zum Ausüben einer Kraft auf das Haar aufweist.

11. Haarbehandlungssystem gemäß einem der Ansprüche 1 bis 10,
wobei die Steuerung der Haarbehandlung ferner auf einem vom Nutzer vorab ausgewählten Zielzustand basiert.

12. Haarbehandlungssystem gemäß Anspruch 11,
wobei die Eingabevorrichtung einen berührungsempfindlichen Bildschirm und/oder eine Tastatur und/oder eine Maus und/oder ein Mikrofon aufweist, und/oder wobei die Ausgabevorrichtung einen Bildschirm und/oder einen Lautsprecher aufweist.

13. Verfahren zum kosmetischen Behandeln von Haaren eines Nutzers, aufweisend:
vor einem Behandeln oder während des Behandelns der Haare mittels eines Haarbehandlungssystems gemäß einem der Ansprüche 1 bis 12 Erfassen eines Haarschädigungsgrads mittels des mindestens einen Zustandssensors; und
Steuern oder Regeln einer Haarbehandlung basierend auf dem empfangenen erfassten Haarschädigungsgrads, wobei die Haarbehandlung ein Aufbringen des Haar-Behandlungsmittels und eine mechanische und/oder thermische Behandlung aufweist.

14. Verfahren gemäß Anspruch 13, ferner aufweisend:
während der Haarbehandlung, Erfassen eines Haarbehandlungsparameters mittels eines Haarbehandlungssensors.

15. Verfahren gemäß einem der Ansprüche 13 oder 14, ferner aufweisend:
Übermitteln des erfassten Haarschädigungsgrads an die elektronische Schaltkreisvorrichtung.

## Claims

1. A hair treatment system comprising:
a sensor device having at least one condition sensor for detecting a degree of hair damage; and
an electric hair treatment device comprising:
an application device for applying a hair treatment agent to the hair;
a hair treatment unit; and
an electronic circuit device,
wherein the electronic circuit device is coupled to the sensor device in order to receive the degree of hair damage, and
wherein the electronic circuit device is designed to control the hair treatment on the basis of the received detected degree of hair damage,
wherein the hair treatment comprises application of the hair treatment agent by means of the application device and mechanical and/or thermal treatment by means of the hair treatment unit,
wherein the sensor device comprises at least one sensor which is designed to detect an environmental parameter.

2. The hair treatment system according to claim 1,
wherein the condition sensor is further designed to detect an environmental parameter, and wherein the electronic circuit device is coupled to the sensor device in order to receive the environmental parameter and/or a hair treatment parameter, and
wherein the electronic circuit device is designed to control the hair treatment on the basis of the received detected environmental parameter and/or the hair treatment parameter.

3. The hair treatment system according to claim 1 or 2,
wherein the hair treatment device comprises a straightening iron or a curling wand.

4. The hair treatment system according to one of claims 1 to 3,
wherein the at least one condition sensor comprises at least one of a group of condition sensors, the group comprising:
a camera for capturing an image using visible light, UV light and/or near infrared light in order to determine the degree of hair damage;
a microscope for determining the degree of hair damage and/or a hair thickness;
a spectrometer for capturing a spectrum of visible light, UV light and/or near infrared light in order to determine the degree of hair damage;
an acoustic sensor for determining the degree of hair damage;
a temperature sensor for determining an ambient temperature;
a humidity sensor; and
a wind sensor.

5. The hair treatment system according to claim 4, wherein the camera is further designed to capture an image using visible light, UV light and/or near infrared light in order to determine a curliness, a hair color, a hair thickness and/or a hair density, and
the spectrometer is further designed to capture a spectrum of visible light, UV light and/or near-infrared light in order to determine a hair amino acid content and/or a hair moisture content.

6. The hair treatment system according to one of claims 1 to 5,
wherein the sensor device and the hair treatment device are separate devices.

7. The hair treatment system according to one of claims 1 to 5,
wherein at least part of the sensor device and the hair treatment device form an integrated unit.

8. The hair treatment system according to one of claims 1 to 7,
wherein the hair treatment system is designed to receive a, preferably predicted, environmental parameter from an external source.

9. The hair treatment system according to one of claims 1 to 8,
wherein the hair treatment device comprises at least one
controlled or regulated element for controlling or regulating the hair treatment.

10. The hair treatment system according to claim 9,
wherein the controlled or regulated element comprises a metering device for the hair treatment agent and/or, as the hair treatment unit, a heating device and/or a motor for exerting a force on the hair.

11. The hair treatment system according to any of claims 1 to 10,
wherein the control of the hair treatment is further based on a target condition selected in advance by the user.

12. The hair treatment system according to claim 11,
wherein the input device comprises a touch-sensitive screen and/or a keyboard and/or a mouse and/or a microphone, and/or
wherein the output device comprises a screen and/or a speaker.

13. A method for the cosmetic treatment of the hair of a user, comprising:
detecting a degree of hair damage by means of the at least one condition sensor before a treatment or during the treatment of the hair by means of a hair treatment system according to one of claims 1 to 12; and
controlling or regulating a hair treatment on the basis of the received detected degree of hair damage, wherein the hair treatment comprises application of the hair treatment agent and mechanical and/or thermal treatment.

14. The method according to claim 13, further comprising:
detecting a hair treatment parameter by means of a hair treatment sensor during the hair treatment.

15. The method according to one of claims 13 or 14, further comprising:
transmitting the detected degree of hair damage to the electronic circuit device.

## Revendications

1. Système de traitement capillaire, présentant :
un dispositif capteur comportant au moins un capteur d'état permettant de détecter un degré d'endommagement des cheveux ; et
un dispositif électrique de traitement capillaire, présentant :
un dispositif d'application permettant d'appliquer un agent de traitement capillaire sur les cheveux ;
une unité de traitement capillaire ; et
un dispositif de circuit électronique,
dans lequel le dispositif de circuit électronique est couplé au dispositif capteur afin de recevoir le degré d'endommagement des cheveux, et
dans lequel le dispositif de circuit électronique est configuré pour commander le traitement capillaire sur la base du degré d'endommagement des cheveux détecté reçu, le traitement capillaire présentant une application de l'agent de traitement capillaire au moyen du dispositif d'application et un traitement mécanique et/ou thermique au moyen de l'unité de traitement capillaire,
dans lequel le dispositif capteur présente au moins un capteur configuré pour détecter un paramètre ambiant.

2. Système de traitement capillaire selon la revendication 1,
dans lequel le capteur d'état est en outre configuré pour détecter un paramètre ambiant et dans lequel le dispositif de circuit électronique est couplé au dispositif capteur afin de recevoir le paramètre ambiant et/ou un paramètre de traitement capillaire, et
dans lequel le dispositif de circuit électronique est configuré pour commander le traitement capillaire sur la base du paramètre ambiant détecté reçu et/ou du paramètre de traitement capillaire.

3. Système de traitement capillaire selon la revendication 1 ou 2,
dans lequel le dispositif de traitement capillaire présente un fer à lisser ou un fer à friser.

4. Système de traitement capillaire selon l'une des revendications 1 à 3,
dans lequel l'au moins un capteur d'état présente au moins l'un parmi un groupe de capteurs d'état, le groupe présentant :
une caméra permettant d'enregistrer une photo au moyen de la lumière visible, de la lumière UV et/ou de la lumière proche infrarouge afin de déterminer le degré d'endommagement des cheveux ;
un microscope permettant de déterminer le degré d'endommagement des cheveux et/ou une épaisseur de cheveux ;
un spectromètre permettant d'enregistrer un spectre de lumière visible, de lumière UV et/ou de lumière proche infrarouge afin de déterminer le degré d'endommagement des cheveux ;
un capteur acoustique permettant de déterminer le degré d'endommagement des cheveux ;
un capteur de température permettant de déterminer une température ambiante ;
un capteur d'humidité de l'air ; et
un capteur de vent.

5. Système de traitement capillaire selon la revendication 4, dans lequel la caméra est en outre configurée pour enregistrer une photo au moyen de la lumière visible, de la lumière UV et/ou de la lumière proche infrarouge afin de déterminer une frisure, une couleur de cheveux, une épaisseur de cheveux et/ou une densité de cheveux,
le spectromètre étant en outre configuré pour enregistrer un spectre de lumière visible, de lumière UV et/ou de lumière proche infrarouge afin de déterminer une teneur des cheveux en acides aminés et/ou une teneur des cheveux en humidité.

6. Système de traitement capillaire selon l'une des revendications 1 à 5,
dans lequel le dispositif capteur et le dispositif de traitement capillaire sont des dispositifs séparés.

7. Système de traitement capillaire selon l'une des revendications 1 à 5,
dans lequel au moins une partie du dispositif capteur et du dispositif de traitement capillaire forment une unité intégrée.

8. Système de traitement capillaire selon l'une des revendications 1 à 7,
dans lequel le système de traitement capillaire est configuré pour recevoir un paramètre ambiant, de préférence prédit, d'une source externe.

9. Système de traitement capillaire selon l'une des revendications 1 à 8,
dans lequel le dispositif de traitement capillaire présente au moins un élément commandé ou régulé permettant de commander ou de réguler le traitement capillaire.

10. Système de traitement capillaire selon la revendication 9,
dans lequel l'élément commandé ou régulé présente un dispositif de dosage destiné à l'agent de traitement capillaire et/ou, en tant qu'unité de traitement capillaire, un dispositif de chauffage et/ou un moteur permettant d'exercer une force sur les cheveux.

11. Système de traitement capillaire selon l'une des revendications 1 à 10,
dans lequel la commande du traitement capillaire est en outre basée sur un état cible sélectionné à l'avance par l'utilisateur.

12. Système de traitement capillaire selon la revendication 11,
dans lequel le dispositif de saisie présente un écran tactile et/ou un clavier et/ou une souris et/ou un microphone, et/ou dans lequel le dispositif de sortie présente un écran et/ou un haut-parleur.

13. Procédé de traitement cosmétique des cheveux d'un utilisateur, présentant :
la détection d'un degré d'endommagement des cheveux au moyen de l'au moins un capteur d'état avant le traitement ou pendant le traitement des cheveux au moyen d'un système de traitement capillaire selon l'une des revendications 1 à 12 ; et
la commande ou la régulation d'un traitement capillaire sur la base du degré d'endommagement des cheveux détecté reçu, le traitement capillaire présentant une application de l'agent de traitement capillaire et un traitement mécanique et/ou thermique.

14. Procédé selon la revendication 13, présentant en outre :
la détection d'un paramètre de traitement capillaire au moyen d'un capteur de traitement capillaire pendant le traitement capillaire.

15. Procédé selon l'une des revendications 13 ou 14, présentant en outre :
la transmission du degré d'endommagement des cheveux détecté au dispositif de circuit électronique.
